# EUROPÄISCHE PATENTSCHRIFT

(11) **EP 4 081 116 B1**
(45) Veröffentlichungstag und Bekanntmachung des Hinweises auf die Patenterteilung: **27.12.2023**
(21) Anmeldenummer: 21717842.5
(22) Anmeldetag: 08.04.2021
(51) Int. Cl.: A61B 5/11, A61B 5/00

(54) **VERFAHREN UND ANORDNUNG ZUR ERZEUGUNG EINES EKG-SIGNALS**
METHOD AND ARRANGEMENT FOR GENERATING AN ECG SIGNAL
PROCÉDÉ ET AGENCEMENT POUR GÉNÉRER UN SIGNAL ECG

(30) Priorität: 09.04.2020 DE 102020204650; 24.06.2020 DE 102020207845
(43) Veröffentlichungstag der Anmeldung: 02.11.2022
(73) Patentinhaber: Fraunhofer-Gesellschaft zur Förderung der angewandten Forschung e.V., 80686 München (DE)
(72) Erfinder: HÄSCHER, Marian, 18057 Rostock (DE)
(74) Vertreter: Ramrath, Lukas
(86) Internationale Anmeldenummer: PCT/EP2021/059165
(87) Internationale Veröffentlichungsnummer: WO 2021/204939

(56) Entgegenhaltungen:
- WO-A1-2020/064469
- MOJTABA JAFARI TADI ET AL: "A real-time approach for heart rate monitoring using a Hilbert transform in seismocardiograms", PHYSIOLOGICAL MEASUREMENT., Bd. 37, Nr. 11, 28. September 2016 (2016-09-28), Seiten 1885-1909, XP055434150, GB ISSN: 0967-3334, DOI: 10.1088/0967-3334/37/11/1885
- LAURIN ALEXANDRE ET AL: "Accurate and consistent automatic seismocardiogram annotation without concurrent ECG", COMPUTING IN CARDIOLOGY, N/A, 6. September 2015 (2015-09-06), Seiten 25-28, XP032865096, ISSN: 2325-8861, DOI: 10.1109/CIC.2015.7408577 ISBN: 978-1-4799-0884-4 [gefunden am 2016-02-16]
- LUU LOC ET AL: "Using Moving Average Method to Recognize Systole and Diastole on Seismocardiogram without ECG Signal", 2018 40TH ANNUAL INTERNATIONAL CONFERENCE OF THE IEEE ENGINEERING IN MEDICINE AND BIOLOGY SOCIETY (EMBC), IEEE, 18. Juli 2018 (2018-07-18), Seiten 3796-3799, XP033429345, DOI: 10.1109/EMBC.2018.8513297 [gefunden am 2018-10-26]
- HAESCHER MARIAN ET AL: "Transforming Seismocardiograms Into Electrocardiograms by Applying Convolutional Autoencoders", ICASSP 2020 - 2020 IEEE INTERNATIONAL CONFERENCE ON ACOUSTICS, SPEECH AND SIGNAL PROCESSING (ICASSP), IEEE, 4. Mai 2020 (2020-05-04), Seiten 4122-4126, XP033792787, DOI: 10.1109/ICASSP40776.2020.9053130 [gefunden am 2020-04-01]

## Beschreibung

Die Erfindung betrifft ein Verfahren und ein System zur Bestimmung eines EKG-Signals.

EKG-Signale werden in einer Vielzahl von Anwendungsfällen benötigt, insbesondere in diagnostischen Anwendungsfällen. Ein normales Oberflächen-EKG kann nicht kontaktlos aufgezeichnet werden. Elektroden werden hierbei benötigt, um die Spannungspotenziale an der Oberfläche des Brustkorbes aufzuzeichnen. Die Langzeitaufzeichnung mit diesen Elektroden kann zu Hautreizungen und Ausschlägen führen. Auch besteht, insbesondere bei solchen Messungen, die Gefahr, dass sich eine Elektrode löst und somit die Qualität des EKG-Signals in unerwünschter Weise beeinträchtigt wird. Manche Patienten (z.B. Frühchen oder Brandopfer) haben zudem eine sehr sensitive und dünne Haut, was ein erhöhtes Risiko von Infektionen und Hautverletzungen bei Kontaktmessung mit sich bringt oder den Einsatz von Elektroden sogar gänzlich verbietet und somit die wichtigen Messungen der Vitaldaten verhindert.

Zudem erlauben die bisherigen EKG-Geräte keine lückenlose und kontinuierliche Messung über einen Zeitraum von 24 Stunden hinaus. Somit können intermittierende kardiologische Pathologien übersehen und somit nicht diagnostiziert und nicht behandelt werden.

Bisher existiert keine medizinisch valide Alternative: Das EKG wird entweder dennoch angelegt und Hautschädigungen und Infektionsrisiko werden nach Abwägung in Kauf genommen oder es wird auf die Messung verzichtet, wodurch Vitaldaten nicht überwacht werden können bzw. keine Diagnostik möglich ist.

Im Falle der kontinuierlichen Erfassung, welche über einen Zeitraum von 24 Stunden hinausgeht, wird im Einzelfall nach Abwägung des Nutzen-Kosten-Verhältnisses auf teure Event Recorder zurückgegriffen, deren Kosten im Regelfall nicht von der Krankenkasse übernommen werden. Event Recorder zeichnen Signale im Falle einer Anomalie mit erhöhter Genauigkeit auf und befinden sich sonst in einem Energiesparmodus.

Auch existieren Einkanal-EKG Lösungen im Bereich der tragbaren Computer (Wearables). Diese bieten eine einfache Aufnahmefunktionalität und eine schnelle Signalanalyse, insbesondere um schwerwiegende Herzkrankheiten zu detektieren. Diese sind jedoch ebenfalls kostenintensiv und müssen - so sie die EKG-Funktion unterstützen - zusätzlich gekauft werden. Die Funktion eines EKGs kann insbesondere nicht einfach per Softwareupdate in alten Geräten nachgerüstet werden. Die medizinische und insbesondere diagnostische Validität der bisherigen Einkanal-EKG Lösungen ist noch nicht gegeben.

Weiter bekannt ist die Erfassung von Seismokardiographiesignalen (SKG-Signal), welches auch als präkordiales Bewegungssignal bezeichnet werden kann. Das Präkordium kann hierbei einen Teil der Brustwand vor dem Herzen bezeichnen. Somit kann das präkordiale Bewegungssignal Informationen über die Bewegung dieses Teils der Brustwand beinhalten. Ein solches Signal beinhaltet insbesondere Informationen über Bewegungen, insbesondere Schwingungen, des Präkordiums, die durch Herzbewegungen bedingt sind. Aus derartigen Signalen können sogar Bewegungen von Herzklappen, z.B. der Aortenklappe oder der Mitralklappe, detektiert und entsprechende Eigenschaften identifiziert werden. Während die elektrischen Reize, die bei der EKG-Untersuchung sichtbar gemacht werden, die elektrischen Reize darstellen, die vor jeder Muskelbewegung innerhalb des Herzzyklus auftreten, repräsentiert das SKG-Signal die resultierenden Bewegungen, die an der präkordialen Position gemessen werden. Bei diesem Ansatz werden zum Beispiel weit verbreitete Trägheitssensoren wie Beschleunigungsmesser oder Gyroskope eingesetzt. Allerdings können auch Druck- oder Radarsensoren verwendet werden.

Ebenfalls bekannt ist die Erfassung von Phonokardiographiesignalen, wobei diese Audiosignale sind, die durch den Empfang von Schallwellen erzeugt werden, wobei die Schallwellen durch Herzbewegungen verursacht sind. Ebenfalls bekannt ist die Erfassung von Ballistokardiographiesignalen, wobei diese die Schwingung des ganzen Körpers erfassen, welche durch die Herzbewegungen verursacht sind. Ballistokardiogramme können am ganzen Körper erfasst werden und sind somit nicht auf einen bestimmten Messpunkt festgelegt. Ein Großteil der Forschung auf dem Gebiet der mobilen und tragbaren Seismokardiographie konzentriert sich auf die Extraktion von Vitalparametern wie Herzfrequenz, Herzfrequenzvariabilität oder Atemfrequenz. Obwohl diese Parameter wertvolle Informationen über den Gesundheitszustand der Benutzer liefern, können Ärzte viel mehr Informationen aus der Rhythmologie und Morphologie des Elektrokardiogramms (EKG) extrahieren.

Ferner bekannt sind Verfahren des maschinellen Lernens, auch in der Kardiologie. Mehrere bekannte Verfahren wenden konvolutionale Autoencoder an, um Gesundheitsdaten zu komprimieren, indem die Komplexität oder das Rauschen in biologischen Signalen reduziert wird, wie für EEG- und EKG-Signale gezeigt wurde.

Insbesondere für die Erkennung von Krankheiten in EKG-Signalen haben sich neuronale Netze als ein leistungsfähiges Werkzeug erwiesen, z.B. zur Detektion von Vorhofflimmern, zur automatisierten Erkennung von Myokardinfarkten sowie zur Erkennung von Arrhythmien.

Neben der Anwendung von neuronalen Netzen zur Analyse von EKG-Daten gibt es eine Reihe von Publikationen, die das maschinelle Lernen auf Signale von anderen Sensortypen anwenden. CNNs (Convolutional Neuronale Netze, faltendes neuronales Netz) können zur Schätzung der Herzfrequenzen von PPG-Sensoren (Photoplethysmografie -Sensoren) oder zur automatischen Erkennung von Herz-Kreislauf-Erkrankungen aus SKG-Daten verwendet werden.

Aus dem Stand der Technik bekannt ist die WO 2014/036436 A1, die eine Vorrichtung und ein Verfahren zum Überwachen des Herzens eines Patienten offenbart. Diese Druckschrift offenbart ein Mobiltelefon mit einem Beschleunigungssensor. Weiter offenbart ist, dass eine entsprechende Vorrichtung auch eine Vielzahl von Elektroden umfassen kann, die in das Mobiltelefon integriert oder an diesem angeordnet sind. Die Vorrichtung kann auf der Brust des Patienten angeordnet werden, um elektrische Signale und Brustvibrationen, die durch einen Herzschlag bedingt sind, zu erfassen. Bei den Messungen kann ein SKG (Seismokardiogramm) und ein EKG (Elektrokardiogramm) erzeugt werden.

Weiter sind im Stand der Technik eine Vielzahl von Lösungen bekannt, die die zeitlich parallele Erfassung eines SKG und eines EKG ermöglichen, beispielsweise aus der EP 2 704 634 A1, der EP 3 135 194 A1, der US 2018/360338 A1 und der WO 2018/231444 A2. Die WO 2019/138327 A1 offenbart ebenfalls ein tragbares EGK-Gerät, welches zusätzlich einen SKG-Sensor umfassen kann. Auch die EP 3 461 401 A1 offenbart eine Vorrichtung zur Erfassung von EGK-Signalen und SKG-Signalen.

Weiter bekannt sind Klassifikationsverfahren zur Klassifizierung eines Zustands eines Fahrzeuginsassen in Abhängigkeit von biometrischen Daten. So offenbart die DE 10 2019 201 695 A1 ein neuronales Netz, welches in einer Fahrzeugkomponente verwendet wird, um den Stresspegel oder Erregungspegel eines Fahrzeuginsassen zu bestimmen. Die US 2019/0088373 A1 offenbart den Einsatz von künstlicher Intelligenz und maschinellem Lernen im Gesundheitswesen.

Die WO 2020/064469 A1 offenbart ein Verfahren zum Bestimmen von kardiophysiologischen Merkmalen für ein Lebewesen.

Es stellt sich daher das technische Problem, ein Verfahren und ein System zur Erzeugung eines EKG-Signals zu schaffen, welche eine genaue und möglichst zuverlässige Erzeugung des EKG-Signals ermöglicht, wobei insbesondere eine kontaktierende Anordnung von Elektroden mit den vorhergehend erläuterten Nachteilen vermieden wird und eine zuverlässige Langzeiterfassung von einem EKG-Signal ermöglicht wird.

Die Lösung des technischen Problems ergibt sich durch die Gegenstände mit den Merkmalen der unabhängigen Ansprüche. Weitere vorteilhafte Ausgestaltungen der Erfindung ergeben sich durch die Gegenstände mit den Merkmalen der Unteransprüche.

Vorgeschlagen wird ein Verfahren zur Erzeugung eines EKG-Signals (Elektrokardiographie-Signals), wobei mindestens ein herzbewegungsinduziertes Signal erfasst wird. Ein herzbewegungsinduziertes Signal kann ein Signal bezeichnen, dass durch Herzbewegungen verursacht wird. Es ist auch möglich, dass mehrere herzbewegungsinduzierte Signale erfasst werden, insbesondere auch Signale verschiedenen Typs. Dies wird nachfolgend noch erläutert. Ein herzbewegungsinduziertes Signal ist erfindungsgemäß ein SKG-Signal (Seismokardiographie-Signal). Auch beschrieben wird, dass es ein PKG-Signal (Phonokardiographie-Signal) oder ein BKG-Signal (Ballistokardiographie-Signal) sein kann. Dieses herzbewegungsinduzierte Signal kann durch eine geeignete Erfassungseinrichtung erzeugt werden. So kann das SKG-Signal von einer geeigneten SKG-Erfassungseinrichtung, das PKG-Signal von einer geeigneten PKG-Erfassungseinrichtung und das BKG-Signal von einer geeigneten BKG-Erfassungseinrichtung erzeugt werden.

Eine solche SKG-Erfassungseinrichtung kann beispielsweise mindestens einen Beschleunigungssensor, z.B. einen MEMS-Beschleunigungssensor, insbesondere ein MEMS-Gyroskop, oder einen Radarsensor, insbesondere einen Doppler-Radarsensor, umfassen. Wie vorhergehend erläutert, enthält bzw. kodiert das SKG-Signal Informationen über Herzbewegungen. Solche Beschleunigungssensoren können uniaxiale oder triaxiale piezoelektische Beschleunigungssensoren oder MEMS-Beschleunigungssensoren, triaxiale MEMS-Beschleunigungssensoren oder Gyroskope, Laser-Doppler-Vibrometer, Mikrowellen-Doppler-Radarsensoren oder eine so genannte Airbourne ultrasound surface motion camera (AUSMC) sein. Eine PKG-Erfassungseinrichtung kann insbesondere ein Mikrofon umfassen, insbesondere ein Mikrofon eines mobilen Endgeräts wie z.B. eines Mobiltelefons oder ein Lasermikrofon. Eine BKG-Erfassungseinrichtung kann z.B. mindestens einen Drucksensor umfassen, z.B. einen als Lastzelle ausgebildeten Drucksensor.

Weiter wird das mindestens eine erfasste herzbewegungsinduzierte Signal in mindestens ein EKG-Signal transformiert. Beispielhafte Transformationsprozesse werden nachfolgend noch näher erläutert. Wie nachfolgend noch näher erläutert ist es auch vorstellbar, dass mindestens eine herzbewegungsinduzierte Signal in mehrere EKG-Signale zu transformieren. Auch ist es - wie nachfolgend noch näher erläutert- auch möglich, dass mehrere erfasste herzbewegungsinduzierte Signale in ein oder mehrere EKG-Signale transformiert werden.

So wurde in überraschender Weise erkannt, dass ein herzbewegungsinduziertes Signal und ein EKG-Signal einen bezüglich der Herzaktivität vergleichbaren Informationsgehalt besitzen, da auch EKG-Signale Informationen über Herzbewegungen enthalten bzw. kodieren. Umgekehrt enthält ein herzbewegungsinduziertes Signal also auch Informationen über elektrische Aktivitäten des Herzens. Da herzbewegungsinduzierte Signale ohne eine entsprechende Verarbeitung für die Nutzer regelmäßig unverständlich sind, weil diese in der Regel im Klinik- und Praxisalltag insbesondere für eine Diagnose nicht verwendet und ihre Interpretation in der Regel nicht Teil der Ausbildung als Arzt ist, kann durch die Transformation ein in der Regel für einen größeren Personenkreis aussagekräftiges EKG-Signal erzeugt werden, wodurch die medizinische Anwendbarkeit z.B. zu Diagnosezwecken steigt. Ferner erfordern EKG-Signale die vorhergehend erläuterte mechanische Kontaktierung der Haut des Patienten, um eine zuverlässige Erzeugung zu ermöglichen. Dies ist bei der Erfassung von herzbewegungsinduzierten Signalen in vorteilhafter Weise nicht zwingend notwendig.

Vorzugsweise wird also das herzbewegungsinduzierte Signal berührungslos erfasst, also ohne mechanische Kontaktierung eines Patienten durch einen entsprechenden Sensor. Z.B. kann dies erfolgen, indem die Erfassungseinrichtung beabstandet von den Patienten angeordnet ist, beispielsweise in einer Matratze, auf der der Patient liegt oder in einem Sitz, in dem der Patient sitzt. Umfasst die Erfassungseinrichtung z.B. einen Radarsensor, so ist es nur erforderlich, die Erfassungseinrichtung derart anzuordnen, dass der Patient bzw. ein Brustbereich des Patienten im Erfassungsbereich des Radarsensors angeordnet ist.

Es ist allerdings auch möglich, dass das herzbewegungsinduzierte Signal durch einen Sensor erfasst wird, der zur Erfassung den Patienten mechanisch kontaktiert bzw. in oder an dem Patienten angeordnet ist. So ist es möglich, dass die Erfassungseinrichtung in einen Schrittmacher, insbesondere in einen ratenadaptiven Schrittmacher, integriert ist. Ein Schrittmacher kann eine solche Erfassungseinrichtung, insbesondere eine als Beschleunigungssensor ausgebildete Erfassungseinrichtung, umfassen, um einen Takt des Herzschlags eines Patienten in Abhängigkeit des von der Erfassungseinrichtung erfassten Signals einzustellen, z.B. um diesen an den aktuellen Bewegungszustand sowie Pulsbedarf anzupassen. Um dies zu ermöglichen werden Aktivitäten in Abhängigkeit von Ausgangssignalen der Beschleunigungssensoren erkannt und z.B. bei steigender Belastung der Takt des Herzschlags entsprechend erhöht (z.B. bei einem Wechsel vom Gehen zu Treppensteigen). Die hierfür eingesetzten Beschleunigungssensoren können zudem eingesetzt werden, um ein herzbewegungsinduziertes Signal zu erfassen.

Ein von einer solchen Erfassungseinrichtung erfasstes Signal kann dann z.B. zu einer Recheneinrichtung übertragen werden, z.B. drahtlos über geeignete Verfahren zur Datenübertragung, wobei die Recheneinrichtung dann die Transformation durchführt. Diese (externe) Recheneinrichtung kann z.B. eine Recheneinrichtung eines mobilen Endgeräts sein. Alternativ ist vorstellbar, dass der Schrittmacher eine Recheneinrichtung umfasst, wobei diese dann die Transformation durchführt. Eine solche Recheneinrichtung des Schrittmachers kann in Form eines eingebetteten Systems in diesen integriert sein. Beispielsweise kann die Recheneinrichtung als integrierte Schaltung ausgebildet sein, welche speziell zur Durchführung der Transformation ausgebildet ist. Diese integrierte Schaltung kann z.B. die Funktionalität eines neuronalen Netzes bereitstellen.

Die Verwendung von einer Erfassungseinrichtung, die in einen Herzschrittmacher integriert ist, ermöglicht in vorteilhafter Weise die Nutzung von bereits vorhandenen und dicht am Herzen angeordneten Sensoren, was eine gute Signalqualität der herzbewegungsinduzierten Signale bedingt. Dies wiederum verbessert die Messgenauigkeit und somit auch die Genauigkeit des erfindungsgemäß erzeugten EKG-Signals. Weiter wird aufgrund der erweiterten Nutzung eines bereits zertifizierten Herzschrittmachers auch eine einfache Zertifizierung eines Systems zur Erzeugung eines EKG-Signals als Medizinprodukt ermöglicht, welches die Erfassungseinrichtung des Herzschrittmachers umfasst.

Durch die Transformation wird also das mindestens eine herzbewegungsinduzierte Signal, welches z.B. präkordiale Bewegungen, von diesen Bewegungen verursachte Schallwellen oder Ganzkörperbewegungen repräsentiert, in ein Signal transformiert, welches elektrische Potentiale repräsentiert bzw. abbildet. Die Transformation in ein EKG-Signal kann eine direkte Transformation sein. Auch kann die Transformation mehrere Teiltransformationen umfassen, wobei z.B. durch eine erste Teiltransformation das herzbewegungsinduzierte Signal in ein Zwischensignal und in einer weiteren Teiltransformation das Zwischensignal in das EKG-Signal transformiert wird. Es ist selbstverständlich möglich, dass auch mehr als zwei Teiltransformationen durchgeführt werden. Durch das vorgeschlagene Verfahren ergibt sich in vorteilhafter Weise eine einfache und zuverlässige Erzeugung eines EKG-Signals, die insbesondere, aber nicht zwingend, berührungslos, also ohne mechanische Kontaktierung der Haut durch eine Erfassungseinrichtung, erfolgen kann. Ferner ermöglicht das vorgeschlagene Verfahren eine zuverlässige Langzeiterfassung von EKG-Signalen, insbesondere über eine Zeitdauer von mehr als 24 Stunden, da herzbewegungsinduzierte Signale problemlos über einen derartigen Zeitraum aufgezeichnet und dann transformiert werden können, insbesondere weil das Problem des ungewollten Ablösens von Elektroden vermieden wird.

Ferner ist es möglich, das beanspruchte Verfahren in vorteilhafter Weise auf existierenden Geräten, die eine zur Erfassung von herzbewegungsinduzierten Signalen geeignete Erfassungseinrichtung aufweisen, zu implementieren und somit nachzurüsten, womit diese Geräte in die Lage versetzt werden, ein EKG-Signal zu erzeugen. Beispielsweise umfassen Mobiltelefone in der Regel Beschleunigungssensoren. Diese können genutzt werden, um SKG-Signale zu erzeugen, beispielsweise indem ein Mobiltelefon auf der Brust eines Patienten platziert und Ausgangssignale des Beschleunigungssensors erfasst werden. Diese Ausgangssignale können dann durch die vorgeschlagene Transformation in ein EKG-Signal transformiert werden. Auch kann ein Mikrofon eines Mobiltelefons genutzt werden, um PKG-Signale zu erzeugen.

Weiter ist das mindestens eine herzbewegungsinduzierte Signal ein SKG-Signal. Hierdurch ergibt sich in vorteilhafter Weise eine zuverlässige Bereitstellung eines EKG-Signals, da SKG-Signale zuverlässig erzeugt werden können. Nicht erfindungsgemäß kann das herzbewegungsinduzierte Signal ein PKG-Signal sein. Da dieses ein breites Frequenzspektrum umfasst ergibt sich in vorteilhafter Weise eine genaue Erzeugung eines EKG-Signals.

Ebenfalls nicht erfindungsgemäß kann das herzbewegungsinduzierte Signal ein BKG-Signal sein. Da dieses am ganzen Körper gemessen werden kann, ergibt sich in vorteilhafter Weise eine flexible Erfassung und somit Erzeugung eines EKG-Signals.

Es ist vorstellbar, dass mehrere, insbesondere verschiedene, herzbewegungsinduzierte Signale erfasst werden, z.B. mehrere SKG-Signale, mehrere BKG-Signale oder mehrere PKG-Signale. Auch können mindestens zwei verschiedene Signale der Signalmenge umfassend SKG-, PKG- und BKG-Signal erfasst werden, wobei das mindestens eine EKG-Signal dann durch eine Transformation dieser verschiedenen Signale in das mindestens eine EKG-Signal erzeugt wird. Auch ist es vorstellbar, dass aus den verschiedenen herzbewegungsinduzierten Signalen ein fusioniertes herzbewegungsinduziertes Signal erzeugt und dieses dann in mindestens ein EKG-Signal transformiert wird.

Erfindungsgemäß wird das mindestens eine herzbewegungsinduzierte Signal in mehrere kanalspezifische Signale eines mehrkanaligen EKG transformiert. Auch ist es vorstellbar, dass ausgewählte, aber nicht alle, kanalspezifischen Signale oder aber alle kanalspezifischen Signale eines mehrkanaligen EKG durch die Transformation des mindestens einen herzbewegungsinduzierten Signals bestimmt wird/werden. So können z.B. aus einem oder mehreren SKG-Signal(en), einem oder mehreren PKG-Signal(en), einem oder mehreren BGK-Signalen oder mindestens zwei verschiedenen Signalen der Signalmenge umfassend SKG-, PKG- und BKG-Signal mehrere kanalspezifische Signale eines EKG bestimmt werden. Das durch Transformation bestimmte kanalspezifische Signal eines mehrkanaligen EKG repräsentiert/simuliert hierbei das in/an einem vorbestimmten Körperbereich durch eine entsprechend angeordnete Elektrode abgeleitete EKG-Signal. Das mehrkanalige EKG kann z.B. ein 6-Kanal- oder ein 12-Kanal-EKG sein. Hierdurch erhöht sich in vorteilhafter Weise eine Gebrauchsfähigkeit der derart erzeugten EKG-Signale, da insbesondere die Erzeugung von einem mehrkanaligen EKG eine einfachere oder bessere Analyse und somit Diagnose ermöglicht.

In einer weiteren Ausführungsform wird die Transformation mittels eines Modells durchgeführt, welches durch maschinelles Lernen erzeugt wurde. Der Begriff maschinelles Lernen umfasst oder bezeichnet hierbei Verfahren zur Bestimmung des Modells basierend auf Trainingsdaten. So ist es möglich, das Modell durch Verfahren zum überwachten Lernen zu bestimmen, wobei hierzu die Trainingsdaten, also ein Trainingsdatensatz, Eingabedaten und Ausgabedaten umfassen. Als Eingabedaten können hierbei herzbewegungsinduzierte Signale bereitgestellt werden, wobei als Ausgabedaten die zu diesen herzbewegungsinduzierten Signalen korrespondierenden EKG-Signale bereitgestellt werden. Insbesondere können Ein- und Ausgabedaten solcher Trainingsdaten erzeugt werden, indem zeitgleich herzbewegungsinduzierte Signale und EKG-Signale erzeugt werden, wobei diese zeitgleich erzeugten Daten dann die Ein- und Ausgabedaten für das Training bilden. Verfahren und Vorrichtung zur zeitgleichen Erzeugung solcher Daten sind aus dem Stand der Technik bekannt, der in der Beschreibungseinleitung erläutert wurde. So kann das Modell hierbei den Zusammenhang zwischen Seismokardiogramm, Ballistokardiogramm bzw. Phonokardiogramm und dem Elektrokardiogramm lernen. Solche Verfahren zum überwachten Lernen sind dem Fachmann bekannt. Es ist auch vorstellbar, dass zur Bestimmung des Modells Verfahren des unüberwachten Lernens angewendet werden.

Nach der Erstellung des Modells, also nach der Trainingsphase, kann das derart parametrisierte Modell in der so genannten Inferenzphase verwendet werden, um aus Eingabedaten in Form von herzbewegungsinduzierten Signalen dann die zu bestimmenden EKG-Signale zu erzeugen, also die vorgeschlagene Transformation durchzuführen. Hierdurch ergibt sich eine zuverlässige und qualitativ hochwertige Erzeugung von EKG-Signalen. Es ist möglich, dass das Modell nutzer- bzw. patientenunspezifisch und/oder erfassungseinrichtungsunspezifisch bestimmt wird, wobei das derart bestimmte Modell dann genutzt wird, um die Transformation für einen spezifischen Nutzer und/oder eine spezifische Erfassungseinrichtung durchzuführen. Dies kann bedeuten, dass das Modell nicht individuell für einen bestimmten Nutzer und/oder für eine bestimmte Erfassungseinrichtung bestimmt wird, aber in der Inferenzphase dann für einen individuellen Nutzer und/oder eine individuelle Erfassungseinrichtung genutzt werden kann.

Es ist also möglich, dass das Modell nicht für jeden Nutzer und/oder jede Erfassungseinrichtung neu trainiert werden muss. Es kann insbesondere einmalig, vorzugsweise mit einem geeignet großen Datensatz, trainiert (Trainingsphase) und danach als Modell nutzer- und/oder erfassungseinrichtungsunabhängig, z.B. für alle Nutzer, eingesetzt (Inferenzphase) werden. Somit ergibt sich in vorteilhafter Weise, dass eine Anwendbarkeit des Verfahrens verbessert wird, da insbesondere nicht für jeden Nutzer und/oder jede Erfassungseinrichtung ein spezifisches Training erfolgen muss. Z.B. kann das gleiche Modell zur Transformation von Signalen genutzt werden, die von verschiedenen Erfassungseinrichtungen erzeugt werden. Hierbei umfasst der geeignete Datensatz vorzugsweise Daten, die für mindestens eine vorbestimmte Anzahl von verschiedenen kranken oder gesunden Personen und/oder für mindestens eine vorbestimmte Anzahl von Physiologien und/oder für mindestens eine vorbestimmte Anzahl von verschiedenen Erkrankungen erzeugt wurden. Selbstverständlich kann es aber erforderlich sein, das Modell mit Eingangsdaten gleicher Charakteristik zu trainieren, also nur mit SKG-Signalen, PKG-Signalen oder BKG-Signalen, wobei jedoch verschiedene Erfassungseinrichtungen oder verschiedene Konfigurationen einer Erfassungseinrichtung zur Erfassung dieser Signale gleicher Charakteristik genutzt werden können. Selbstverständlich ist es aber auch möglich, dass das Modell nutzer- und/oder erfassungseinrichtungsspezifisch bestimmt wird.

Geeignete mathematische Algorithmen für das maschinelle Lernen umfassen: Decision Tree-basierte Verfahren, Ensemble Methods (z.B. Boosting, Random Forrest) -basierte Verfahren, Regression-basierte Verfahren, Bayes'sche Methoden (z.B. Bayesian Belief Networks)-basierte Verfahren, Kernel Methoden (z.B. Support Vector Machines)-basierte Verfahren, Instance- (z.B. k-Nearest Neighbour)-basierte Verfahren, Association Rule Learning-basierte Verfahren, Boltzmann Maschine-basierte Verfahren, Artificial Neural Networks (z.B. Perceptron) -basierte Verfahren, Deep Learning (z.B. Convolutional Neural Networks, Stacked Autoencoders) -basierte Verfahren, Dimensionality Reduction-basierte Verfahren, Regularization Methods-basierte Verfahren.

Zum Trainieren z.B. eines neuronalen Netzes benötigt man regelmäßig eine große Menge an Trainingsdaten, um eine gewünschte Qualität der Transformation zu gewährleisten. Die Menge der Trainingsdaten kann abhängig von Faktoren wie der Komplexität des zugrundeliegenden Problems, der benötigten Genauigkeit und der angestrebten Anpassungsfähigkeit des zu trainierenden Netzes sein. Der Anwendungsbereich, also die Domäne, in der das Netz zum Einsatz kommen soll, ist oftmals das wichtigste Element in der Bestimmung dieser Faktoren und somit der Bestimmung der Trainingsdatenmenge. Mit entsprechendem Vorwissen über die Domäne ist es möglich zum Training des Netzes Daten vorzubereiten, die zu einer schnelleren Konvergenz zur optimalen Lösung führen, bzw. eine solche Konvergenz überhaupt erst ermöglichen und somit weniger Trainingsdaten benötigen.

Das vorgeschlagene Verfahren kommt in medizinischem Umfeld zum Einsatz. Somit ist eine hohe Genauigkeit wünschenswert. Hinzu kommt eine vergleichsweise hohe Komplexität, da sich EKG-Signale und herzbewegungsinduzierte Signale auf Grund der unterschiedlichen Sensorik zu deren Erfassung voneinander unterscheiden. Dies bedingt aber in der Regel eine große Datenmenge zum Trainieren eines neuronalen Netzes. Ein möglicher Schritt, um die benötigte Menge an Daten zu reduzieren, besteht in der Filterung der Trainingsdaten, insbesondere der Eingabedaten und/oder der Ausgabedaten. Insbesondere können Ein- und Ausgabedaten eines Trainingsdatensatzes erzeugt werden, indem zeitgleich herzbewegungsinduzierte Signale und EKG-Signale erzeugt und dann vor dem Training gefiltert werden. Somit verringert sich der Speicherbedarf als auch die benötigte Rechenzeit und/oder -leistung zur Bestimmung/Erzeugung des Modells. So ist es möglich, die Trainingsdaten mit einem Filter zu filtern, insbesondere einem Bandpassfilter, z.B. einem Butterworthfilter, um hochfrequente sowie niederfrequente Anteile in den Trainingsdaten abzuschwächen. Beispielsweise kann eine erste, untere Grenzfrequenz eines Bandpassfilters 0.5 Hz und eine weitere, obere Grenzfrequenz 200 Hz betragen. Auch vorstellbar ist die Nutzung von Hoch- und/oder Tiefpassfiltern oder anderen Filter (z.B. Polynomfilter), die entsprechenden ungewollten Frequenzen aus den Trainingsdaten herausfiltern. Alternativ können die erzeugten Signale aber auch ungefiltert zum Training genutzt werden.

In einer weiteren Ausführungsform wird zur Erzeugung des Modells eine Fehlerfunktion zur Bestimmung einer Abweichung zwischen einem durch Transformation bestimmten EKG-Signal und einem Referenz-EKG-Signal ausgewertet, wobei bei der Auswertung der Fehlerfunktion verschiedene Signalabschnitte des durch Transformation bestimmten EKG-Signals und/oder des Referenz-EKG-Signals und/oder der Abweichung (des Abweichungssignals) verschieden gewichtet werden. Somit kann also eine EKG-Signalspezifische Fehlerfunktion verwendet werden. Das Referenz-EKG-Signal kann eine Grundwahrheit repräsentieren und kann beispielsweise ein parallel zu den Eingangsdaten (also einem herzbewegungsinduzierten Signal) erfasstes EKG-Signal sein, welches mit einer bekannten, z.B. elektrodenbasierten, EKG-Erfassungseinrichtung erfasst wurde. Die Fehlerfunktion wird genutzt, um eine Abweichung zwischen dem Ergebnis der Transformation, also dem durch die Transformation bestimmten EKG-Signal, und der Grundwahrheit zu bestimmen bzw. zu quantifizieren. Diese Abweichung beeinflusst dann die Bestimmung, insbesondere das Training, des Modells für die Transformation durch maschinelles Lernen, insbesondere die Bestimmung eines neuronalen Netzes, wobei das Modell z.B. derart angepasst wird, dass die Abweichung reduziert wird. Hierbei kann als Abweichung z.B. eine mittlere quadratische Abweichung oder eine mittlere absolute Abweichung bestimmt werden.

Es ist möglich, dass zur Bestimmung der Abweichung verschiedene Signalabschnitte des durch Transformation bestimmten EKG-Signals oder des Referenz-EKG-Signals verschieden und alle Signalabschnitte des verbleibenden Signals gleich gewichtet werden. Vorzugsweise werden zur Bestimmung der Abweichung alle Signalabschnitte des durch Transformation bestimmten EKG-Signals und alle Signalabschnitte des Referenz-EKG-Signals gleich gewichtet, wobei jedoch verschiedene Abschnitte des die Abweichung repräsentierenden Signals verschieden gewichtet werden. Ein gewichteter Abschnitt im Abweichungssignal kann ein Abschnitt sein, der zu einem vorbestimmten (relevanten) Abschnitt in dem durch Transformation bestimmten EKG-Signals und/oder im Referenz-EKG-Signal zeitlich korrespondiert. Die verschiedene Gewichtung verschiedener Signalabschnitte in mindestens einem der genannten Signale kann eine Qualität des Modells und somit auch die Signalqualität des durch Transformation bestimmten EKG-Signals in vorteilhafter Weise verbessern. Die verschiedene Gewichtung verschiedener Signalabschnitt ermöglicht es insbesondere charakteristische und somit relevante Abschnitte des EKG-Signals höher zu gewichten als weniger relevante. Relevante EKG-Signalabschnitte können von einem Experten identifiziert werden, beispielsweise durch eine Auswahl von Signalabschnitten mittels einer Eingabeeinrichtung. Alternativ ist es jedoch auch vorstellbar, eine automatisierte Detektion von relevanten Signalabschnitten durchzuführen, beispielsweise über geeignete Detektionsverfahren, die z.B. Abschnitte mit vorbestimmten Signaleigenschaften identifizieren. In solchen Detektionsverfahren kann beispielsweise eine Phasortransformation durchgeführt werden. In diesem Fall können Abschnitten mit vorbestimmten Signaleigenschaften vorbestimmte Gewichte zugeordnet sein. Ein relevanter Abschnitt in einem Signal kann ein P-Wellen-Signalabschnitt, ein QRS-Komplex-Signalabschnitt und/oder ein T-Wellenabschnitt sein.

In einer weiteren Ausführungsform wird die Transformation mittels eines neuronalen Netzes durchgeführt. Beispielsweise kann das neuronale Netz als Autoencoder oder als faltendes neuronales Netz (Convolutional Neural Network, CNN) oder als RNN (Recurrent Neural Network) oder als LSTM-Netz (long short-term memory network) oder als neuronales Transformer-Netz oder als eine Kombination aus mindestens zwei der erwähnten Netze ausgebildet sein. Ein solches neuronales Netz, insbesondere das als Autoencoder ausgebildete neuronale Netz, kann hierbei mittels der vorhergehend erläuterten Trainingsdaten trainiert werden, wobei dann nach dem Training die Transformation eines erfassten herzbewegungsinduzierten Signals in das EKG-Signal durchgeführt werden kann. Hierbei bietet die Ausbildung des neuronalen Netzes als Autoencoder in vorteilhafter Weise, dass der für die Transformation benötigte Rechenaufwand gering ist, wodurch die Transformation in einfacher Weise durch eingebettete Systeme und tragbare Endgeräte wie z.B. Mobilfunktelefone zuverlässig und zeitlich schnell durchgeführt werden kann.

Die Ausbildung als CNN erlaubt in vorteilhafter Weise eine Reduktion der Komplexität des Netzes und ist damit geeignet für Geräte mit geringer Rechenleistung. Dies betrifft sowohl die Trainingsphase als auch die Inferenzphase. Auch ergibt sich in vorteilhafter Weise, dass die zum Training benötigte Zeitdauer bei CNN kurz ist, insbesondere kürzer als bei LSTM-Netzen, die ebenfalls im Vergleich höhere Rechenleistungen benötigen. Die Ausbildung als LSTM-Netz weist jedoch eine besonders gute Eignung für die Analyse von Zeitreihen auf, da ihre Architektur den Bezug zu zeitlichen Abhängigkeiten berücksichtigt. Somit ergibt sich in vorteilhafter Weise eine hohe Qualität der Transformation und des damit bestimmten EKG-Signals.

In einer alternativen Ausführungsform wird die Transformation mittels eines vorbestimmten mathematischen Modells oder mittels einer vorbestimmten Transformationsfunktion durchgeführt. Diese kann beispielsweise durch einen Nutzer vorbestimmt sein. Insbesondere ist es möglich, mathematische Modelle zur Transformation von herzbewegungsinduzierten Signalen in EKG-Signale geeignet zu parametrisieren. Hierdurch ergibt sich in vorteilhafter Weise eine alternative zuverlässige und zeitlich schnelle Erzeugung von EKG-Signalen.

In einer weiteren Ausführungsform wird das mindestens eine herzbewegungsinduzierte Signal berührungslos erfasst. Werden mehrere solche Signale erfasst, so kann/können genau ein, mehrere, aber nicht alle oder aber alle Signale berührungslos erfasst werden. Dies und entsprechende Vorteile wurden vorhergehend erläutert.

In einer weiteren Ausführungsform wird das mindestens eine herzbewegungsinduzierte Signal vor der Transformation gefiltert und dann das gefilterte herzbewegungsinduzierte Signal in ein EKG-Signal transformiert. Die Filterung kann insbesondere eine Hoch- oder Bandpass- oder Bandstoppfilterung sein. Ein entsprechender Filter zur Durchführung der Filterung kann insbesondere ein Butterworth oder Polynomfilter sein. Ist die Filterung eine Hochpassfilterung, so kann eine Grenzfrequenz des Hochpassfilters beispielsweise in einem Bereich von 5 Hz bis 8 Hz liegen, um zuverlässig Auswirkungen von Bewegungsartefakten auf das herzbewegungsinduzierte Signal zu reduzieren. Ist die Filterung eine Bandpassfilterung, so kann eine erste Grenzfrequenz beispielsweise in einem Bereich von 5 Hz (einschließlich oder ausschließlich) bis 8 Hz (einschließlich oder ausschließlich) und eine weitere Grenzfrequenz in einem Bereich von 30 Hz (einschließlich oder ausschließlich) bis 35 Hz (einschließlich oder ausschließlich) liegen, um ebenfalls die Auswirkung von Bewegungsartefakten zuverlässig zu reduzieren, die beispielsweise außerhalb des Bereichs von 8 Hz bis 30 Hz liegen. Die Filterung kann insbesondere durch Butterworth-Filter oder Polynomfilter durchgeführt werden. Hierdurch ergibt sich in vorteilhafter Weise eine genauere Bestimmung des EKG-Signals, insbesondere auch dann, wenn sich der Patient während der Erfassung des herzbewegungsinduzierten Signals bewegt.

In einer weiteren Ausführungsform wird das mindestens eine herzbewegungsinduzierte Signal von einer Erfassungseinrichtung eines Geräts erzeugt. Beispielhafte Erfassungseinrichtungen wurden vorhergehend bereits erläutert. Das Gerät bezeichnet hierbei eine Einheit, die die Erfassungseinrichtung umfasst. So kann das Gerät beispielsweise ein Mobiltelefon oder ein Tablet-PC sein. Selbstverständlich sind jedoch auch andere Ausführungsformen eines solchen Geräts vorstellbar. Weiter wird die Transformation durch eine Recheneinrichtung des Geräts durchgeführt. Mit anderen Worten umfasst das Gerät sowohl die Erfassungseinrichtung als auch die Recheneinrichtung. Eine Recheneinrichtung kann hierbei als Mikrocontroller oder integrierte Schaltung ausgebildet sein oder eine solche(n) umfassen. So ist es möglich, die Transformation oder eine Teiltransformation durch ein programmierbares oder fest verdrahtetes Bauteil, insbesondere einen Chip (z.B. ASIC, FPGA), durchzuführen. Ein solches Bauteil kann dann in Alleinstellung oder als Teil eines system-in-package (SiP) die Transformation durchführen. Auch ist es möglich, die Mittel zur Durchführung der Transformation, z.B. als SoC (systemon-a-chip), direkt in einen Sensor zur Erfassung des herzbewegungsinduzierten Signals (z.B. MEMS-Beschleunigungssensor) oder in ein anderes elektronisches Bauteil zu integrieren. Hierdurch ergibt sich in vorteilhafter Weise eine zentralisierte Erfassung und Erzeugung von EKG-Signalen, beispielsweise auf einem Endgerät, insbesondere einem mobilen Endgerät.

Das Gerät kann neben den erläuterten Mitteln zur Signalverarbeitung auch Mittel zur Signalspeicherung, Mittel zur Signalübertragung und Mittel zur Anzeige umfassen. Allerdings kann es auch möglich sein, dass das Gerät keines oder nicht alle der erläuterten Mittel umfasst. In diesem Fall kann das erfasste herzbewegungsinduzierte Signal an ein weiteres Gerät übertragen werden, welches eines oder mehrere weitere Mittel umfasst. Das derart erzeugte EKG-Signal kann also auch visualisiert werden, beispielsweise durch eine Anzeigeeinrichtung des Geräts. Auch kann das EKG-Signal gespeichert werden, beispielsweise durch eine Speichereinrichtung des Geräts. Weiter ist es möglich, das EKG-Signal von dem Gerät an ein externes System zu übertragen, beispielsweise über eine geeignete Kommunikationseinrichtung des Geräts.

Alternativ wird das herzbewegungsinduzierte Signal von der Erfassungseinrichtung an eine geräteexterne Recheneinrichtung übertragen, wobei die Transformation von dieser geräteexternen Recheneinrichtung durchgeführt wird. Die geräteexterne Recheneinrichtung kann insbesondere eine Servereinrichtung oder die Recheneinrichtung eines weiteren Geräts sein. Auch in diesem Fall kann das herzbewegungsinduzierte Signal visualisiert werden, beispielsweise durch eine Anzeigeeinrichtung des Geräts, wozu die durch die von der geräteexternen Recheneinrichtung durchgeführten Transformation bestimmten EKG-Signale wieder an das Gerät zurückübertragen werden. Selbstverständlich ist es auch möglich, das derart bestimmte EKG-Signal durch eine geräteexterne Anzeigeeinrichtung zu visualisieren. Hierzu kann das EKG-Signal an das entsprechende weitere Gerät zur Anzeige übertragen werden. Weiter kann das derart bestimmte EKG-Signal gespeichert oder weiterverarbeitet werden, beispielsweise durch die geräteexterne Speicher- bzw. Recheneinrichtung oder eine weitere (geräteexterne) Speicher- bzw. Recheneinrichtung.

Die geräteexterne Recheneinrichtung kann hierbei eine Servereinrichtung eines Netzwerks, insbesondere des Internets, sein oder bilden. Insbesondere kann die geräteexterne Recheneinrichtung Teil einer Servereinrichtung sein, die cloudbasierte Dienste anbietet. Die Übertragung an die geräteexterne Recheneinrichtung kann vorzugsweise drahtlos, beispielsweise durch geeignete Übertragungsverfahren, erfolgen. Selbstverständlich ist es aber auch möglich, die Übertragung drahtgebunden auszugestalten. Hierdurch ergibt sich in vorteilhafter Weise, dass die Recheneinrichtung eines Geräts, welches auch die Erfassungseinrichtung umfasst, nicht durch die Transformation überlastet wird. Insbesondere ist es somit möglich, die Erfassung des herzbewegungsinduzierten Signals durch Geräte durchzuführen, die vergleichsweise geringe Rechenleistung bereitstellen, wodurch dann entsprechende Transformation und gegebenenfalls Weiterverarbeitung durch andere Recheneinrichtungen mit im Vergleich höherer Rechenleistung durchgeführt werden kann.

In einer weiteren Ausführungsform wird das mindestens eine herzbewegungsinduzierte Signal von einer Erfassungseinrichtung eines Geräts erzeugt und das durch Transformation bestimmte EKG-Signal auf einer Anzeigeeinrichtung des Geräts oder auf einer externen Anzeigeeinrichtung, beispielsweise einer Anzeigeeinrichtung eines weiteren Geräts, dargestellt. So ist es beispielsweise möglich, dass das herzbewegungsinduzierte Signal von dem Gerät an eine geräteexterne Recheneinrichtung übertragen und dort die Transformation durchgeführt wird, wobei dann das derart bestimmte EKG-Signal an ein weiteres Gerät, beispielsweise ein weiteres Mobilfunktelefon, übertragen wird und dann auf dessen Anzeigeeinrichtung dargestellt wird. Auch kann das EKG-Signal wieder an das Gerät zurückübertragen und durch dessen Anzeigeeinrichtung dargestellt werden. Auch können die EKG-Signale durch eine Anzeige in einem Browser angezeigt werden, insbesondere falls die geräteexterne Recheneinrichtung eine Servereinrichtung oder ein Teil davon ist. Hierdurch ist es möglich, dass eine Fernüberwachung auf Basis der erfindungsgemäß erzeugten EKG-Signale durchgeführt werden kann.

In einer weiteren Ausführungsform wird vor der Transformation des mindestens einen herzbewegungsinduzierten Signals ein Funktionstest einer Erfassungseinrichtung durchgeführt, wobei das herzbewegungsinduzierte Signal nur dann transformiert wird, wenn eine Funktionsfähigkeit detektiert wird. Eine Funktionsfähigkeit kann beispielsweise detektiert werden, wenn die Erfassungseinrichtung ein zeitlich veränderliches Ausgangssignal erzeugt. Wird ein zeitlich konstantes Ausgangssignal erzeugt oder weicht das Ausgangssignal nicht mehr als ein vorbestimmtes Maß von einem konstanten Ausgangssignal ab, so kann eine fehlende Funktionsfähigkeit detektiert werden. Alternativ oder kumulativ kann eine Funktionsfähigkeit detektiert werden, wenn das Ausgangssignal Eigenschaften aufweist, die mehr als ein vorbestimmtes Maß von vorbestimmten Rauscheigenschaften abweichen, insbesondere Eigenschaften von weißem Rauschen. Ist dies der Fall, so kann eine Funktionsfähigkeit detektiert werden. Ist dies nicht der Fall, so kann eine fehlende Funktionsfähigkeit detektiert werden. Eine fehlende Funktionsfähigkeit kann auch detektiert werden, wenn eine Abtastrate des Ausgangssignals von einer Soll-Abtastrate abweicht und/oder eine Quantisierung des Ausgangssignals von zulässigen Quantifizierungswerten abweicht. Bei einer fehlenden Funktionsfähigkeit kann keine Transformation durchgeführt werden. Hierdurch ergibt sich in vorteilhafter Weise, dass die Transformation nur dann durchgeführt wird, wenn von einer Funktionsfähigkeit der Erfassungseinrichtung ausgegangen werden kann. Hierdurch wird ein Energieverbrauch bei der Durchführung des Verfahrens reduziert.

Alternativ oder kumulativ wird vor der Transformation des mindestens einen herzbewegungsinduzierten Signals eine Signalgüte des erfassten Signals bestimmt, wobei das herzbewegungsinduzierte Signal nur dann transformiert wird, wenn eine die Signalgüte größer als ein oder gleich einem vorbestimmtes/n Maß ist. Eine Signalgüte kann beispielsweise ein Signal-zu-Rausch-Verhältnis oder eine dieses Verhältnis repräsentierende Größe sein. Ist dieses Verhältnis größer als ein vorbestimmtes Maß, so kann die Transformation durchgeführt werden. Auch kann eine Signalgüte größer als ein oder gleich einem vorbestimmten Maß sein, wenn eine Abweichung zwischen einem vorbestimmten Referenz-Signalverlauf und einem erfassten Signalverlauf in einem Abschnitt des herzbewegungsinduzierten Signals kleiner als ein oder gleich einem vorbestimmten Maß ist. Dies kann auch als sogenannter Templatevergleich bezeichnet werden. Hierbei kann eine klassische Signalform eines herzbewegungsinduzierten Signals, also der Referenz-Signalverlauf, bestimmt und abgespeichert werden. Dann kann mit dem Fachmann bekannte Verfahren eine Abweichung zwischen dem Signalverlauf des erfassten herzbewegungsinduzierten Signals mit dem Referenz-Signalverlauf bestimmt werden.

Auch kann eine Signalgüte über geeignete Modelle wie z.B. neuronale Netze bestimmt werden. Trainingsdaten für derartige Modelle können erzeugt werden, indem einem herzbewegungsinduzierten Signal ein die Signalgüte repräsentierendes Gütemaß zugeordnet wird, z.B. durch einen Nutzer oder (teil-)automatisiert. Diese Zuordnung kann auch als Annotation bezeichnet werden. Hierbei bilden das herzbewegungsinduzierte Signal die Eingangsdaten und das Gütemaß die Ausgangsdaten des Trainingsdatensatzes. Solche Trainingsdaten können insbesondere erzeugt werden, indem herzbewegungsinduzierte Signale in verschiedenen Raumlagen der Erfassungseinrichtung, insbesondere relativ zum Herzen, mit verschiedenen SNR, unter verschiedenen Umgebungsbedingungen, in verschiedenen Bewegungszuständen des Patienten usw. erzeugt und annotiert werden.

Es ist weiter möglich, dass ein solches Modell, insbesondere ein neuronales Netz, zur Bestimmung der Signalgüte auch zur Filterung der Trainingsdaten für die Bestimmung des durch maschinelles Lernen erzeugten Modells für die Transformation genutzt wird. Hierbei werden also nur solche herzbewegungsinduzierten Signale als Eingangsdaten für das Training des Modells zur Transformation genutzt, für die die Signalgüte höher als ein vorbestimmtes Maß ist. Durch die Auswertung der Signalgüte als Bedingung für die Durchführung der Transformation kann in vorteilhafter Weise sichergestellt werden, dass eine zuverlässige und qualitativ hochwertige Transformation erfolgt.

Es ist auch möglich, dass zusätzlich zur Signalgüte eine qualitätsmindernde Ursache über geeignete Modelle wie z.B. neuronale Netze bestimmt wird. Trainingsdaten für derartige Modelle können erzeugt werden, indem einem herzbewegungsinduzierten Signal die qualitätsmindernde Ursache zugeordnet wird, z.B. durch einen Nutzer oder (teil- )automatisiert. Diese Zuordnung kann auch als Annotation bezeichnet werden. Hierbei bilden das herzbewegungsinduzierte Signal die Eingangsdaten und die Ursache die Ausgangsdaten des Trainingsdatensatzes. Qualitätsmindernde Ursachen können beispielsweise das Vorhandensein von Artefakten, die Anordnung der Erfassungseinrichtung in für die Erfassung ungünstigen Raumlagen, insbesondere relativ zum Herzen, und/oder das Vorhandensein von ungünstigen Umgebungs- oder Bewegungsbedingungen sein.

Kann derart eine qualitätsmindernde Ursache bestimmt werden, so kann der Nutzer, beispielsweise über eine Anzeigeeinrichtung, über die Ursache informiert werden. Zusätzlich kann dem Nutzer eine Handlungsempfehlung zur Behebung der Ursache gegeben werden.

Weiter alternativ oder kumulativ wird vor der Transformation des mindestens einen herzbewegungsinduzierten Signals eine Lage, also eine räumliche Position und/oder Orientierung, der Erfassungseinrichtung relativ zum Herz bestimmt, wobei das herzbewegungsinduzierte Signal nur dann transformiert wird, wenn die Lage einer vorbestimmten Lage entspricht oder weniger als ein vorbestimmtes Maß von dieser abweicht. Es ist beispielsweise möglich, dass das herzbewegungsinduzierte Signal nur dann vorbestimmte Signaleigenschaften aufweist, wenn die Lage einer vorbestimmten Lage entspricht oder weniger als ein vorbestimmtes Maß von dieser abweicht. Somit können Signaleigenschaften des herzbewegungsinduzierten Signals bestimmt und mit den vorbestimmten Signaleigenschaften verglichen werden. Ist die Abweichung geringer als ein vorbestimmtes Maß, so entspricht die Lage der vorbestimmten Lage oder weicht weniger als ein vorbestimmtes Maß von dieser ab.

Es ist auch möglich, dass die Lage über geeignete Modelle wie z.B. neuronale Netze bestimmt wird. Trainingsdaten für derartige Modelle können erzeugt werden, indem einem herzbewegungsinduzierten Signal die Lage zugeordnet wird, z.B. durch einen Nutzer oder (teil-)automatisiert. Diese Zuordnung kann auch als Annotation bezeichnet werden. Hierbei bilden das herzbewegungsinduzierte Signal die Eingangsdaten und die Lage die Ausgangsdaten des Trainingsdatensatzes. Solche Trainingsdaten können insbesondere erzeugt werden, indem herzbewegungsinduzierte Signale in verschiedenen Raumlagen der Erfassungseinrichtung, insbesondere relativ zum Herzen, erzeugt und entsprechend annotiert werden. Kann eine Lage bestimmt werden, so kann der Nutzer, beispielsweise über eine Anzeigeeinrichtung, über die Lage, insbesondere ihre Korrektheit, informiert werden.

Zusätzlich kann dem Nutzer eine Handlungsempfehlung zur Veränderung der Lage gegeben werden, wenn diese mehr als das vorbestimmte Maß von der vorbestimmten Lage abweicht.

Durch die Bestimmung der Lage als Bedingung für die Durchführung der Transformation kann in vorteilhafter Weise sichergestellt werden, dass eine zuverlässige und qualitativ hochwertige Transformation erfolgt. So kann beispielsweise vermieden werden, dass eine Erfassungseinrichtung zur Erfassung des herzbewegungsinduzierten Signals nicht in korrekter Weise angeordnet ist, beispielsweise ein Beschleunigungssensor nicht auf einer Körperoberfläche aufliegt, und somit eine Qualität des durch Transformation bestimmten EKG-Signals reduziert ist.

Es ist vorstellbar, dass zur Bestimmung der Funktionsfähigkeit und/oder der Signalgüte und/oder der Lage die zur Transformation vorgesehenen herzbewegungsinduzierte Signale ausgewertet werden, wobei diese zur Transformation verwendet werden, wenn eine Funktionsfähigkeit detektiert wird und/oder die Signalgüte größer als das oder gleich dem vorbestimmte/n Maß ist und/oder die Lage nicht mehr als ein vorbestimmtes Maß von der vorbestimmten Lage abweicht. Alternativ kann die Funktionsfähigkeit und/oder die Signalgüte und/oder die Lage auf Basis von nicht zur Transformation vorgesehenen herzbewegungsinduzierte Signale bestimmt werden, wobei dann eine weitere Erfassung des herzbewegungsinduzierten Signals zur Transformation durchgeführt wird, wenn eine Funktionsfähigkeit detektiert wird und/oder die Signalgüte größer als das oder gleich dem vorbestimmte/n Maß ist und/oder die Lage nicht mehr als ein vorbestimmtes Maß von der vorbestimmten Lage abweicht.

Das EKG-Signal kann insbesondere das EKG-Signal eines Menschen sein, also ein Signal für/in humanmedizinische Anwendungen. Allerdings kann das Verfahren auch zur Erzeugung eines EKG-Signals eines Tieres angewendet werden, also zur Erzeugung eines Signals für/in tiermedizinische Anwendungen. So ermöglicht eine insbesondere elektrodenlose EKG-Erfassung bei Tieren in vorteilhafter Weise eine große Reduktion von Stress bei Tieren, bei denen ein EKG z.B. zu diagnostischen Zwecken erfasst werden soll.

So können bei Tieren, insbesondere Pferden oder Hunden, 24-Stunden-Holter-EKG erfasst werden, was aber aufgrund des notwendigen Arztbesuchs und der anschließenden Verkabelung des Tieres einen großen Stressfaktor für die Tiere darstellt. Dies kann insbesondere dann problematisch sein, wenn es sich bei den Pathologien um episodenhafte Erkrankungen handelt, da Stress beispielsweise bei Hunden für eine nicht erkennbare klinische Symptomatik sorgt. Das erfindungsgemäße Verfahren benötigt zur Erzeugung eines EKG-Signals jedoch kein Fachpersonal, insbesondere zum Anlegen der Elektroden, und es ermöglicht eine kontaktlose Messung, insbesondere auch von mehrkanaligen EKG-Signalen.

So kann beispielsweise eine Erfassungseinrichtung in ein Geschirr oder Brustgurt, das/der dem Tier angelegt wird, integriert werden. Somit kann eine solche Sensorik vom Tierhalter selbst gekauft und angelegt werden. So kann z.B. ein Beschleunigungssensor im Geschirr/Brustgurt die herzbewegungsinduzierten Signale des Tieres erfassen und die erläuterte Transformation ermöglichen. Ebenso kann das Verfahren genutzt werden, um von Tierärzten in Routineuntersuchungen eingesetzt zu werden. Da Tiere in der Regel erst sehr spät Symptome einer Erkrankung des Herz-Kreislauf-Systems zeigen, kann man auf diesem Weg bereits Diagnosen in einem früheren Stadium solcher Krankheiten ermöglichen. Der Tierarzt kann dabei durch das Auflegen einer geeigneten Erfassungseinrichtung oder Gerätes mit einer Erfassungseinrichtung, z.B. eines Smartphones, ein Ruhe-EKG des Tieres erfassen, ohne dass dafür eine Vielzahl von Elektroden appliziert werden müssen. Dabei entfallen zudem Nachteile einer Elektrodennutzung am Tier (z.B. Artefakte auf dem EKG-Signal durch Fell, welches die Elektroden stört). Dieses Konzept der Untersuchung lässt sich zudem sowohl auf Haustierfische, z.B. Kois, als auch auf Pferde und Kamele anwenden, was besonders im Leistungssportbereich solcher Tiere interessant ist.

Im Nutztierbereich werden medizinische Überwachungen regelmäßig nach Kosten bzw. Aufwand nur reduziert durchgeführt, z.B. indem ein Tierarzt nach Kohorten diagnostiziert. Jedoch würde eine EKG- Überwachung auch hier wertvolle Informationen bezüglich des Tierwohls für den Arzt bereitstellen (etwa zur Leistungsfähigkeit, Gesundheitsstatus, Stressbewertung, frühzeitige Erkennung bakterieller Infektionen wie Streptokokken). Bisher ist jedoch eine EKG-Überwachung einzelner Tiere mit den gängigen Methoden sehr aufwendig und teuer. Das vorgeschlagene Verfahren bietet eine preiswerte und einfache Möglichkeit der Überwachung, z.B. wenn das herzbewegungsinduzierte Signal berührungslos erfasst wird, z.B. durch die Nutzung von Radarsensoren. Somit können Tiere kontaktlos und somit auch hygienisch überwacht werden. Denkbar wäre diese Überwachung für Nutztiere wie Schweine, Wiederkäuer, aber auch Fische. Auch in der Tierforschung kann das vorgeschlagene Verfahren eingesetzt werden. Anwendbar ist es auch in Zoos und Tierparks, um die Gesundheit der Tiere bei möglichst wenig Stress zu gewährleisten.

Weiter vorgeschlagen wird ein System zur Erzeugung eines EKG-Signals, wobei das System mindestens eine Erfassungseinrichtung zur Erfassung mindestens eines herzbewegungsinduzierten Signals und mindestens eine Recheneinrichtung umfasst. Wie vorhergehend erläutert, kann die Erfassungseinrichtung und die Recheneinrichtung jeweils Teil eines Geräts sein. Allerdings ist es auch vorstellbar, dass die Erfassungseinrichtung und die mindestens eine Recheneinrichtung jeweils Teile voneinander verschiedener Geräte sind. Auch ist vorstellbar, dass das System mehrere Erfassungseinrichtungen zur Erfassung mehrerer herzbewegungsinduzierter Signale umfasst.

Erfindungsgemäß ist das mindestens eine erfasste herzbewegungsinduzierte Signal mittels der Recheneinrichtung in mindestens ein EKG-Signal transformierbar. Hierzu kann es notwendig sein, dass durch die Erfassungseinrichtung erfasste Signal an die Recheneinrichtung zu übertragen, beispielsweise durch Übertragungseinrichtungen.

Das System ermöglicht in vorteilhafter Weise die Durchführung eines Verfahrens zur Erzeugung eines EKG-Signals gemäß einer der in dieser Offenbarung beschriebenen Ausführungsformen mit den entsprechend genannten Vorteilen. Somit ist das System derart konfiguriert, dass ein solches Verfahren mit dem System ausgeführt werden kann.

In einer weiteren Ausführungsform ist die Erfassungseinrichtung in einen Inkubator integriert. Beispielsweise kann die Erfassungseinrichtung in diesem Fall einen Doppler-Radarsensor umfassen und an einer Decke des Inkubators angeordnet sein, insbesondere derart, dass ein Brustbereich des Patienten, der auf einer Matratze des Inkubators liegt, im Erfassungsbereich des Radarsensors angeordnet ist. Alternativ kann die Erfassungseinrichtung einen Beschleunigungssensor umfassen oder als solcher ausgebildet sein, der im/am Boden des Inkubators oder in/an der Matratze des Inkubators angeordnet ist.

Alternativ kann die Erfassungseinrichtung in einem Bett, insbesondere einem Krankenhausbett, angeordnet sein. Ist die Erfassungseinrichtung z.B. als Doppler-Radarsensor ausgebildet, so kann diese unter der Matratze oder über dem Bett, beispielsweise an einem Bettgalgen befestigt, angeordnet sein. Ebenfalls vorstellbar ist die vorhergehend erläuterte Ausbildung der Erfassungseinrichtung als Beschleunigungssensor, der in/an der Matratze oder in/an dem Boden des Bettes angeordnet ist. Auch ist es möglich, die Erfassungseinrichtung als Drucksensor auszubilden, der in/an der Matratze des Bettes angeordnet ist. Weiter alternativ ist die Erfassungseinrichtung in einen Fahrzeugsitz integriert. Hierbei kann eine als Doppler-Radarsensor ausgebildete Erfassungseinrichtung beispielsweise in/an einer Sitzlehne angeordnet sein. Ein als Drucksensor ausgebildete Erfassungseinrichtung kann in/an der Sitzlehne angeordnet sein. Selbiges gilt für eine als Beschleunigungssensor ausgebildete Erfassungseinrichtung. Weiter alternativ ist die Erfassungseinrichtung in einen Herzschrittmacher integriert. Weiter alternativ ist die Erfassungseinrichtung in einen Tierbedarfsgegenstand, z.B. ein Brustgurt, ein Halfter, ein Halsband oder ähnliches, integriert.

Somit wird auch ein System zur Erzeugung eines EKG-Signals beschrieben, welches einen Inkubator umfasst, wobei die Erfassungseinrichtung in/an dem Inkubator oder in/an einer Matratze des Inkubators angeordnet ist. Weiter wird ein System beschrieben, welches ein Bett umfasst, wobei die Erfassungseinrichtung in/an dem Bett oder in/an einer Matratze des Betts angeordnet ist. Weiter wird ein System zur Erzeugung eines EKG-Signals beschrieben, welches zusätzlich einen Fahrzeugsitz umfasst, wobei die Erfassungseinrichtung in/an dem Fahrzeugsitz angeordnet ist. Weiter wird ein System zur Erzeugung eines EKG-Signals beschrieben, welches zusätzlich einen Herzschrittmacher umfasst, wobei die Erfassungseinrichtung in/an dem Herzschrittmacher angeordnet ist. Weiter wird ein System zur Erzeugung eines EKG-Signals beschrieben, welches zusätzlich einen Tierbedarfsgegenstand umfasst, wobei die Erfassungseinrichtung in/an dem Tierbedarfsgegenstand angeordnet ist. Selbstverständlich sind auch weitere Anwendungen vorstellbar. Auch beschrieben wird ein Inkubator, ein Bett, eine Matratze, ein Fahrzeugsitz, ein Herzschrittmacher und ein Tierbedarfsgegenstand, der/die/das zumindest die Erfassungseinrichtung eines solchen Systems umfasst.

Die Erfindung wird anhand von Ausführungsbeispielen näher erläutert. Die Figuren zeigen:
- Fig. 1: eine schematische Darstellung eines erfindungsgemäßen Verfahrens zur Bestimmung eines EKG-Signals,
- Fig. 2: ein schematisches Blockschaltbild eines erfindungsgemäßen Systems zur Erzeugung eines EKG-Signals gemäß einer ersten Ausführungsform,
- Fig. 3: eine schematische Darstellung eines erfindungsgemäßen Systems zur Erzeugung eines EKG-Signals gemäß einer weiteren Ausführungsform,
- Fig. 4: ein schematisches Flussdiagramm eines erfindungsgemäßen Verfahrens,
- Fig. 5: eine schematische Darstellung eines Systems zur Erzeugung eines EKG-Signals gemäß einer weiteren Ausführungsform,
- Fig. 6: eine schematische Darstellung eines Systems zur Erzeugung eines EKG-Signals gemäß einer weiteren Ausführungsform,
- Fig. 7: eine schematische Darstellung eines Systems zur Erzeugung eines EKG-Signals gemäß einer weiteren Ausführungsform,
- Fig. 8: eine schematische Darstellung einer exemplarischen Anwendung des erfindungsgemäßen Verfahrens,
- Fig. 9: eine schematische Darstellung eines Systems zur Erzeugung eines EKG-Signals mit einem Inkubator,
- Fig. 10: eine schematische Darstellung eines Systems zur Erzeugung eines EKG-Signals mit einem Krankenhausbett,
- Fig. 11: eine schematische Darstellung eines Systems zur Erzeugung eines EKG-Signals mit einem Fahrzeugsitz,
- Fig. 12: eine schematische Darstellung eines erfindungsgemäßen Verfahrens in einer weiteren Ausführungsform,
- Fig. 13: eine schematische Darstellung der Erzeugung/des Trainings des in Fig. 12 dargestellten neuronalen Netzes,
- Fig. 14: eine schematische Darstellung synchronisierter EKG- und SKG-Signale,
- Fig. 15: eine schematische Darstellung eines durch Transformation bestimmten EKG-Signals und eines durch Elektroden aufgezeichneten EKG-Signals,
- Fig. 16a: eine schematische Darstellung eines Hundegurts mit einer Erfassungseinrichtung eines Systems zur Erzeugung eines EKG-Signals,
- Fig. 16b: eine schematische Darstellung eines Pferdeholsters mit einer Erfassungseinrichtung eines Systems zur Erzeugung eines EKG-Signals,
- Fig. 17: eine schematische Darstellung eines Schrittmachers mit einem System zur Erzeugung eines EKG-Signals und,
- Fig. 18: eine exemplarische Darstellung von Gewichtungen verschiedener Signalabschnitte.

Nachfolgend bezeichnen gleiche Bezugszeichen Elemente mit gleichen oder ähnlichen technischen Merkmalen.

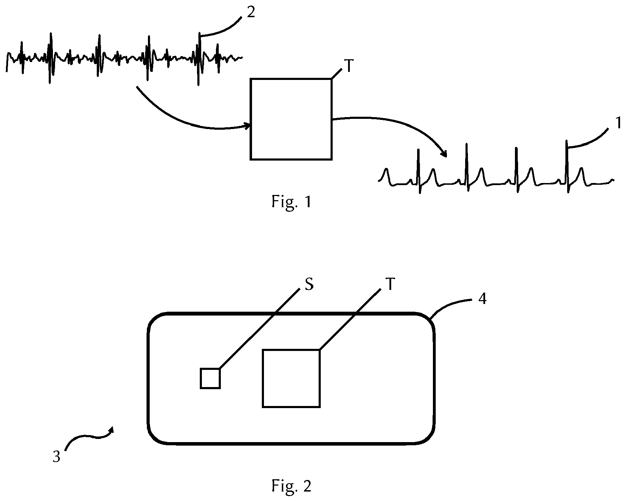

Fig. 1 zeigt eine schematische Darstellung eines Verfahrens zur Erzeugung eines EKG-Signals 1. Hierbei wird ein als SKG-Signal 2 ausgebildetes herzbewegungsinduziertes Signal erfasst. Dies kann mittels einer nachfolgend noch näher erläuterten SKG-Erfassungseinrichtung S erfolgen. Dann wird durch eine Transformationseinrichtung T, die insbesondere als Recheneinrichtung ausgebildet oder eine Recheneinrichtung umfassen kann, das erfasste SKG-Signal 2 in ein EKG-Signal 1 transformiert. Alternativ oder kumulativ kann als herzbewegungsinduziertes Signal auch ein PKG-Signal, z.B. durch eine PKG-Erfassungseinrichtung, erfasst und in ein EKG-Signal 1 transformiert werden. Weiter alternativ oder kumulativ kann als herzbewegungsinduziertes Signal auch ein BKG-Signal, z.B. durch eine BKG-Erfassungseinrichtung, erfasst und in ein EKG-Signal 1 transformiert werden.

Fig. 2 zeigt ein schematisches Blockschaltbild eines Systems 3 zur Erzeugung eines EKG-Signals 1 (siehe Fig. 1). Das System 3 umfasst eine SKG-Erfassungseinrichtung S und mindestens eine Transformationseinrichtung T, die als Recheneinrichtung ausgebildet ist.

Dargestellt ist, dass die SKG-Erfassungseinrichtung und die Transformationseinrichtung Teil eines Geräts 4 sind, beispielsweise eines Mobiltelefons.

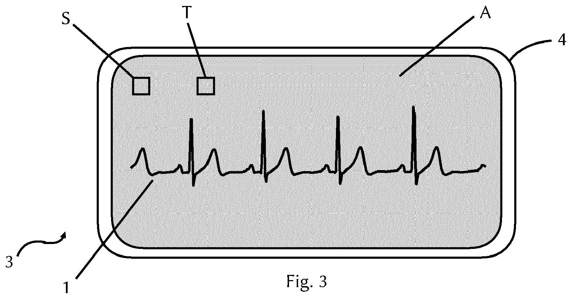

Fig. 3 zeigt eine Darstellung des Systems 3 zur Erzeugung eines EKG-Signals 1 gemäß einer weiteren Ausführungsform. Wie vorhergehend erläutert, umfasst das System 3 eine SKG-Erfassungseinrichtung S und eine als Recheneinrichtung ausgebildete Transformationseinrichtung T. Weiter dargestellt ist eine Anzeigeeinrichtung A, auf der das EKG-Signal 1 visualisiert wird. Hierbei ist dargestellt, dass die SKG-Erfassungseinrichtung S, die Transformationseinrichtung T und die Anzeigeeinrichtung A Teil eines Geräts 4 sind.

Die in Fig. 2 und Fig. 3 dargestellte SKG-Erfassungseinrichtung kann beispielsweise als Beschleunigungssensor, als Drucksensor oder als Radarsensor, insbesondere Doppler-Radarsensor, ausgebildet sein oder einen solchen Sensor umfassen. Auch kann die SKG-Erfassungseinrichtung als Gyroskop ausgebildet sein oder ein solches Gyroskop umfassen.

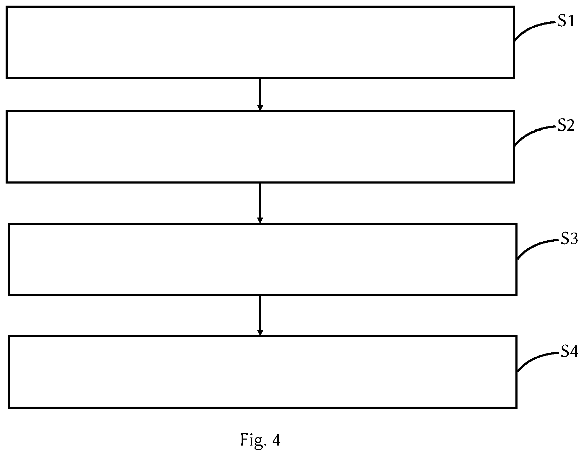

Fig. 4 zeigt ein schematisches Flussdiagramm eines erfindungsgemäßen Verfahrens. Hierbei wird in einem Erfassungsschritt S1 ein SKG-Signal 1 erfasst, insbesondere mittels einer SKG-Erfassungseinrichtung S, die vorhergehend erläutert wurde. In einem optionalen Filterschritt S2 wird das derart erfasste SKG-Signal 2 gefiltert, beispielsweise hochpassgefiltert. Auch kann eine sogenannte Trendbeseitigung im SKG-Signal 2 durchgeführt werden. In einem Transformationsschritt S3, der in der Transformationseinrichtung T durchgeführt werden kann, wird das SKG-Signal in ein EKG-Signal transformiert. Somit kann auch ein Seismokardiogramm in ein Elektrokardiogramm transformiert werden. Der Transformationschritt S3 kann auch mehrere Teiltransformationen umfassen. In einem Nachverarbeitungsschritt S4 wird das derart erzeugte EKG-Signal bzw. das derart erzeugte Elektrokardiogramm gespeichert, an mindestens eine weitere Einrichtung übertragen und/oder visualisiert, beispielsweise auf einer geeigneten Anzeigeeinrichtung A.

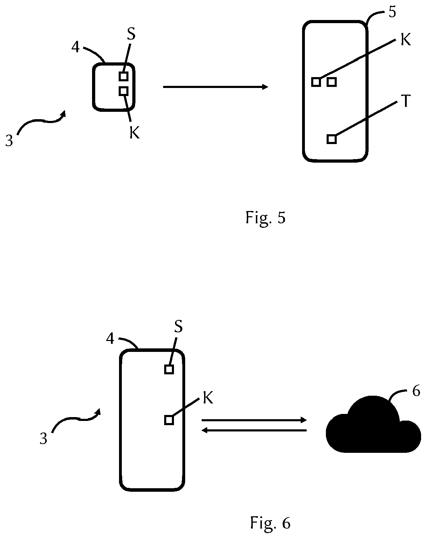

Fig. 5 zeigt eine schematische Darstellung eines Systems 3 zur Erzeugung eines EKG-Signals 1 (siehe Fig. 1) gemäß einer weiteren Ausführungsform. Dargestellt ist ein Gerät 4, welches eine SKG-Erfassungseinrichtung S umfasst. Durch diese SKG-Erfassungseinrichtung S ist ein SKG-Signal 2 (siehe Fig. 1) erfassbar. Weiter umfasst das Gerät eine Kommunikationseinrichtung K zur Datenübertragung zwischen dem Gerät 4 und weiteren Geräten. Durch diese Kommunikationseinrichtung K wird das erzeugte SKG-Signal 1 an ein HUB-Gerät 5 übertragen. Dieses HUB-Gerät 5 weist eine als Recheneinrichtung ausgebildete Transformationseinrichtung T und eine Kommunikationseinrichtung K zum Empfang der übertragenen SKG-Signale auf. Weiter kann durch die HUB-Einrichtung 5 die Transformation des SKG-Signals 2 in das EKG-Signal 1 durchgeführt werden. Es ist dann möglich, dass das derart bestimmte EKG-Signal 1 dann auf einer nicht dargestellten Anzeigeeinrichtung der HUB-Einrichtung 5 zur Anzeige gebracht wird. Auch kann es durch eine nicht dargestellte Speichereinrichtung der HUB-Einrichtung 5 gespeichert oder durch die Kommunikationseinrichtung K weiter übertragen werden.

Fig. 6 zeigt eine weitere Darstellung eines Systems 3 zur Erzeugung eines EKG-Signals 1. Im Unterschied zu der in Fig. 5 dargestellten Ausführungsform werden die von der SKG-Erfassungseinrichtung S erzeugten SKG-Signale 2 über die Kommunikationseinrichtung K an eine Servereinrichtung 6 übertragen, die sogenannte Cloud-basierte Dienste anbietet. Diese Servereinrichtung 6 kann eine nicht dargestellte Transformationseinrichtung T umfassen, die die Transformation der vom Gerät 4 übertragenen SKG-Signale 2 in EKG-Signale 1 durchführt. In Fig. 6 ist dargestellt, dass die transformierten Signale, also die EKG-Signale 1, wieder an das Gerät 4 zurückübertragen werden, wobei diese dann durch die Kommunikationseinrichtung K des Geräts 4 empfangen werden können. Dann kann das derart erhaltene EKG-Signal von dem Gerät 4 gespeichert, weiterverarbeitet oder visualisiert werden, beispielsweise durch eine nicht dargestellte Anzeigeeinrichtung A des Geräts 4. Hierbei ist es möglich, dass mindestens ein Nachbearbeitungsschritt durch die HUB-Einrichtung 5 oder durch die Servereinrichtung 6 durchgeführt wird. Hierbei können einzelne, mehrere, aber nicht alle, oder aber alle der vorhergehend erläuterten Nachbearbeitungsschritte von der HUB-Einrichtung 5 oder der externen Servereinrichtung 6 durchgeführt werden.

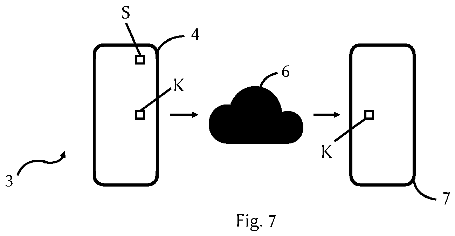

Fig. 7 zeigt eine schematische Darstellung eines Systems 3 zur Erzeugung eines EKG-Signals 1 gemäß einer weiteren Ausführungsform der Erfindung. Im Unterschied zu der in Fig. 6 dargestellten Ausführungsform werden von der SKG-Erfassungseinrichtung S des Geräts 4 erfasste SKG-Signale 2 über die Kommunikationseinrichtung K des Geräts 4 an die Servereinrichtung 6 übertragen, deren Transformationseinrichtung dann die Transformation in EKG-Signale 1 durchführt. Die derart transformierten EKG-Signale 1 werden dann von der Servereinrichtung 6 an ein weiteres Gerät 7 übertragen und dort mittels einer Kommunikationseinrichtung K des weiteren Geräts 7 empfangen. Weiter können die derart erzeugten EKG-Signale 1 dann in einer Speichereinrichtung des weiteren Geräts 7 gespeichert, von einer Recheneinrichtung des weiteren Geräts 7 weiterverarbeitet oder von einer nicht dargestellten Anzeigeeinrichtung des weiteren Geräts 7 angezeigt werden.

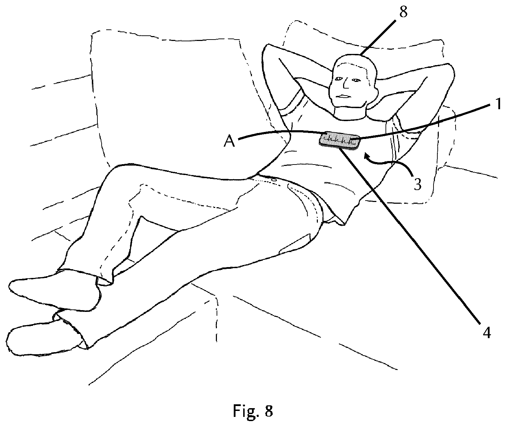

Fig. 8 zeigt eine schematische Anwendung eines Systems 3 (siehe z.B. Fig. 2) zur Erzeugung eines EKG-Signals 1. Hierbei ist ein als Mobilfunktelefon 4 ausgebildetes Gerät, welches eine nicht dargestellte SKG-Erfassungseinrichtung S und eine als Recheneinrichtung ausgebildete Transformationseinrichtung T umfasst, auf einer Brust eines Nutzers/Patienten 8 angeordnet. Es ist selbstverständlich vorstellbar, dass anstelle des Mobilfunktelefons 4 auch ein anderes Gerät mit einer SKG-Erfassungseinrichtung S genutzt wird.

Mittels der SKG-Erfassungseinrichtung S können dann SKG-Signale 2 erzeugt werden, die dann durch die Transformationseinrichtung (nicht dargestellt) des Geräts 4 in EKG-Signale 1 transformiert und dann von einer Anzeigeeinrichtung A des Geräts 4 visualisiert werden.

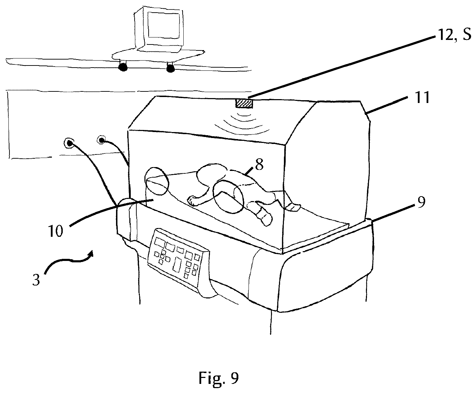

Fig. 9 zeigt eine Darstellung eines Systems 3 zur Erzeugung eines EKG-Signals 1 (siehe Fig. 1) gemäß einer weiteren Ausführungsform. Das System 3 umfasst einen Inkubator 9, wobei ein Patient 8, beispielsweise eine Frühgeburt, auf einer Matratze 10 des Inkubators 9 liegt. Weiter umfasst der Inkubator 9 einen Deckel 11, der den Liegeraum für den Patienten 8 abdeckt. An den Deckel ist eine als ein Doppler-Radarsensor 12 ausgebildete SKG-Erfassungseinrichtung S angeordnet. Hierbei ist dieser Doppler-Radarsensor 12 derart angeordnet, dass ein Brustbereich des Patienten 8 im Erfassungsbereich dieses Sensors 12 liegt. Alternativ wäre es möglich, z.B. eine als Druck- oder Beschleunigungssensor ausgebildete SKG-Erfassungseinrichtung S in/an der Matratze 10 oder in/an einem Boden des Inkubators 9, auf dem die Matratze 10 aufliegt, anzuordnen. Ist der Patient 8 ein Frühgeborenes bzw. ein Neugeborenes, so kann, insbesondere durch geeignete Filterverfahren, ein von Umgebungsartefakten völlig oder in hohem Maße bereinigtes EKG-Signal 1 erzeugt werden, da mit der im Vergleich hohen Herzfrequenz eines Neugeborenen eine zuverlässige Reduktion von Störeinflüssen anderer Personen im Umfeld des Inkubators 9 erreicht werden kann.

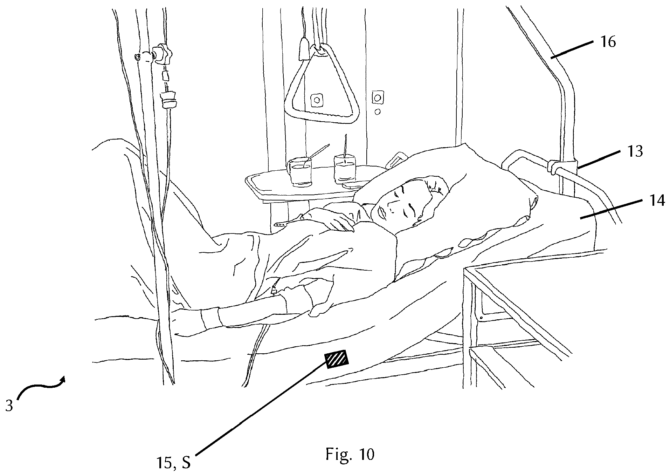

Fig. 10 zeigt eine schematische Darstellung eines Systems 3 zur Erzeugung eines EKG-Signals 1 (siehe Fig. 1) gemäß einer weiteren Ausführungsform. Das System 3 umfasst ein Bett 13 mit einer Matratze 14. Weiter umfasst das System 3 eine als Druck- oder Beschleunigungssensor 15 ausgebildete SKG-Erfassungseinrichtung S, die in/an der Matratze 14 angeordnet ist. Selbstverständlich ist es auch vorstellbar, einen Doppler-Radarsensor zu verwenden, wobei dieser beispielsweise an einem Galgen 16 des Bettes 13 angeordnet werden kann.

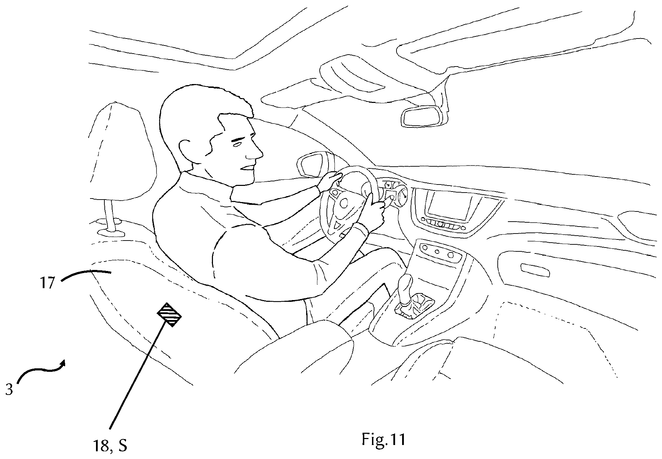

Fig. 11 zeigt eine schematische Darstellung eines Systems 3 zur Erzeugung eines EKG-Signals 1 (siehe Fig. 1) gemäß einer weiteren Ausführungsform. Das System 3 umfasst hierbei einen Fahrzeugsitz 17, wobei in eine Rückenlehne des Fahrzeugsitzes 17 eine als Druck- oder Beschleunigungssensor 18 ausgebildete SKG-Erfassungseinrichtung S, angeordnet ist. Selbstverständlich ist es auch vorstellbar, die SKG-Erfassungseinrichtung S als Doppler-Radarsensor auszubilden und in geeigneter Weise in/an der Rückenlehne oder an einer davon verschiedenen Stelle des Fahrzeugs anzuordnen.

Die in den Figuren 8, 9, 10, 11 dargestellten Ausführungsformen ermöglichen neben der normalen Überwachung von Vitaldaten und der normalen Diagnostik von kardiologischen Pathologien eine günstige, lückenlose sowie elektrodenlose Überwachung und somit auch die Detektion von gegebenenfalls bisher nicht diagnostizierten kardiologischen Pathologien wie z.B. einem intermittierenden Vorhofflimmern.

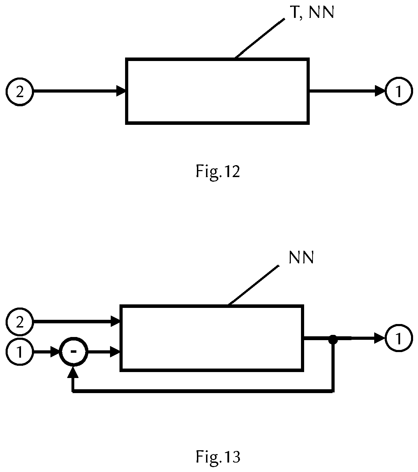

Fig. 12 zeigt eine schematische Darstellung eines erfindungsgemäßen Verfahrens in einer weiteren Ausführungsform. Hierbei ist dargestellt, dass SKG-Signale 2 Eingangsdaten für ein neuronales Netz NN bilden, welches die Transformation von SKG-Signalen in EKG-Signale 1 durchführt. Somit sind die Ausgangssignale des neuronalen Netzes NN die wie vorgeschlagen zu erzeugenden EKG-Signale 1. In diesem Fall ist die Transformationseinrichtung T als neuronales Netz NN ausgebildet, umfasst ein solches bzw. kann Funktionen eines neuronalen Netzes NN ausführen.

Fig. 13 zeigt eine schematische Darstellung der Erzeugung/des Trainings des in Fig. 12 dargestellten neuronalen Netzes NN. Hierbei werden Trainingsdaten in Form von zeitgleich erfassten SKG-Signalen 2 und EKG-Signalen 1 in das neuronale Netz NN eingespeist, wobei Parameter des neuronalen Netzes NN derart angepasst werden, dass eine Abweichung zwischen den vom neuronalen Netz erzeugten EKG-Signalen 1, die Ausgangsdaten des neuronalen Netzes NN sind, und EGK-Signalen des Trainingsdatensatzes minimiert wird. Der Trainingsdatensatz kann aus einer kombinierten Messung von EKG-Signalen, Atmung und Seismokardiogramm resultieren. Ein solcher Datensatz ist z.B. in Form eines öffentlich zugänglicher Datensatz als Teil der Physiobank verfügbar. Für die Erprobung des Verfahrens wurden Daten von 20 (12 männlichen und 8 weiblichen) vermutlich gesunden Probanden verwendet. Das Durchschnittsalter der Probanden betrug 24,4 Jahre (SD ±3,10). Zum Zweck der Datenerfassung wurde ein Biopac MP36 eingesetzt, wobei EKG-Signale 1 über den ersten und zweiten Kanal und SKG-Signale 2 über den vierten Kanal mit einem Beschleunigungsmesser (LIS344ALH, ST Microelectronics) erfasst wurden. Die Probanden wurden gebeten, wach und still in Rückenlage zu liegen. Es wurden drei Arten von Aufnahmen durchgeführt (Basalzustand, fünf Minuten; Hören von klassischer Musik, 50 Minuten; Kontrollzustand, eine Minute). Die Aufzeichnung der EKG-Signale 1 wurde mit einer Bandbreite zwischen 0,05 Hz und 150 Hz durchgeführt, die SGK-Signale 2 wurden mit einer Bandbreite zwischen 0,5 Hz und 100 Hz erfasst. In jedem Kanal wurde mit einer Abtastrate von 5kHz abgetastet.

Folgend wird beispielhaft die Architektur des verwendeten neuronalen Netzes und die Trainingsdaten, einschließlich ihrer Vorverarbeitung, beschrieben, so wie diese für die Erprobung des Verfahrens appliziert wurde.

Insbesondere um das neuronale Netz auf eingebetteten Geräten und intelligenten tragbaren Geräten wie z.B. Telefonen zu betreiben, wurde ein Faltungs-Autoencoder (Convolutional Autoencoder) für das Lernen der SKG-zur-EKG-Transformation verwendet. Der Autoencoder verwendet einen Encoder und einen Decoder mit jeweils vier eindimensionalen Faltungsschichten. Im Encoder folgen auf die Faltungsschichten eine ReLU-Aktivierungsfunktion für die Abbildung von Nichtlinearität und eine Max-Pooling-Schicht für die Reduktion von Rechenaufwand, die zur Reduktion einer Überanpassung und/oder zur Auflösung starrer Spatialrelation dient. Im Encoder beginnt die erste Faltungsschicht mit 128 Filtern mit einer Kernelgröße von 8, mit jeder weiteren Schicht verdoppelt sich die Anzahl der Filter. Der latente Raum (latent space) halbiert die Anzahl der Filter. Im Decoder beginnt der Decoder mit 256 Filtern in der ersten Faltungsschicht. In der zweiten und dritten Schicht halbiert sich die Anzahl der Filter jeweils. Die letzte Faltungsschicht reduziert die Anzahl der Filter von 64 auf 1. Jede Schicht in dem Decoder besteht aus einer Upsampling-Schicht, einer Faltungsschicht und einer ReLU-Aktivierungsfunktion.

SKG- und EKG-Aufzeichnungen des Datensatzes wurden mit einer Abtastrate von 100 Hz neu abgetastet, um diese an die gängige Abtastrate bei der Erfassung der Beschleunigung anzupassen, die typischerweise zwischen 100 Hz und 200 Hz arbeiten. Dies ermöglicht eine langfristige SKG-zu-EKG-Transformation trotz der begrenzten Rechenleistung von eingebetteten Geräten. Das SKG-Signal wurde mit einem 5-30Hz-Bandpass-Butterworth-Filter vierter Ordnung gefiltert. Anschließend wurde das Signal normalisiert (lineare Abbildung zwischen 0 und 1). Eine zusätzliche Filterung der EKG-Signale wurde nicht vorgenommen, da diese bereits vorgefiltert waren.

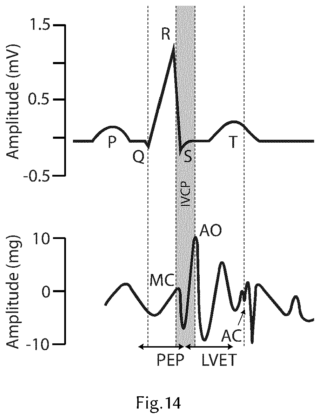

Fig. 14 zeigt eine schematische Darstellung von synchronisierten EKG- und SKG-Signalen, wobei das EKG-Signal in der oberen und das SKG-Signal in der unteren Zeile dargestellt ist.

Vor dem Training wurden die Gewichte der Faltungsschichten des Modells mit einer Glorot-Uniform-Initialisierung vorinitialisiert. Die Verlustfunktion ist durch den mittleren absoluten Fehler gegeben und wird durch den Adam-Optimierer mit Standardparametern und ohne Regularisierungsterm optimiert. Das Label bzw. die Referenz ist ein Ground-Truth-EKG-Signal (EKGGT), so dass der Autoencoder eine Abbildung von SKG-Signalen 2 auf EKGGT-Signale lernt und dann das SKG-Signal 2 in ein durch Transformation bestimmtes EKG-Signal 1 (EKGT) transformiert. In einem nächsten Schritt wird jedes 512 Werte lange SKG-Fenster in das Netz eingespeist. Das Ergebnis des Modells ist ein 512 Werte langes EKGT-Fenster, das über eine Verlustoptimierung an das entsprechende EKGGT-Fenster angepasst wird.

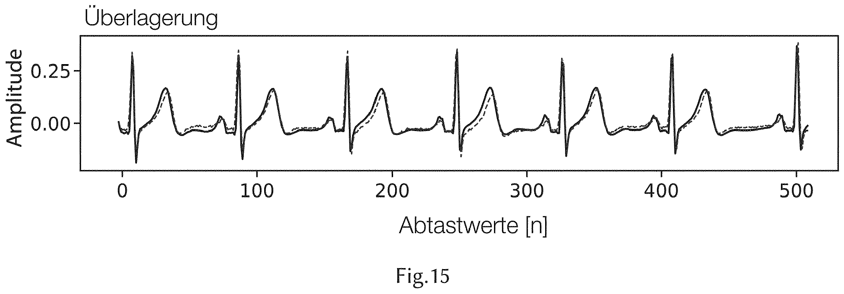

Für das Training wurde eine Schiebefenster-Technik angewendet, um die Anzahl der Proben zu erhöhen und sicherzustellen, dass das Netz die Übergänge zwischen den Fenstern richtig erfasst. Die Wahl einer Fenstergröße von 512 mit einer Überlappung von 87,5% ergab 4.040 nutzbare Fenster für jeden Teilnehmer. Für alle 20 Teilnehmer wird die Eingabe daher zu einem Tensor 20 x 512 x 4040 x 2 umgestaltet. Aufgrund der geringen Anzahl von Probanden wurde eine leave-one-out k-fold Kreuzvalidierung durchgeführt, um die Generalisierungsleistung des Modells zu bewerten. Die Leistung wurde durch Mittelwertbildung der 20 Faltungen berechnet. Um zu veranschaulichen, wie die EKGT- und die EKGGT-Signale aussehen, zeigt Figur 15 das Transformationsergebnis (EKGT-Signal), welches durch eine gestrichelte Linie dargestellt ist, eines 400 Proben langen Segments (Benutzer 10 im Datensatz) mit einer Überlagerung des EKGGT, welches durch eine durchgezogene Linie dargestellt ist. Die Ergebnisse wurden mit drei verschiedenen Arten von Metriken ausgewertet: 1) Bewertung auf Signalebene; 2) Bewertung auf Merkmalsebene; 3) Bewertung durch Domänenexperten.

### Auswertung auf Signalebene

Es wurden Kreuzkorrelationen verwendet, um das EKGGT mit dem EKGT zu vergleichen. Beide Signale korrelieren hoch mit einem Korrelationskoeffizienten von r=0,94. Um die Qualität der Transformationsergebnisse auf Signalebene zu analysieren, wurde ebenfalls eine Reihe von geeigneten EKG-Vergleichswerten einschließlich des mittleren quadratischen Fehlers, des normalisierten mittleren quadratischen Fehlers, des mittleren quadratischen Wurzelfehlers, des normalisierten mittleren quadratischen Wurzelfehlers und der prozentualen mittleren quadratischen Differenz ausgewertet. Außerdem wurde eine "leave-one-out"-Kreuzvalidierung mit allen Probanden durchgeführt und die Mittelwerte und Standardabweichungen für jeden Indikator berechnet. Ergebnisse sind in Tabelle 1 angeführt.

### Bewertung auf Merkmalsebene

Für Vergleiche auf Merkmalsebene wurden aus beiden Signalen zwei wichtige EKG-Merkmale, nämlich die Anzahl der R-Spitzen und die Dauer der QRS-Komplexe, extrahiert.

| **Table 1: Mittelwerte (M) und Standardabweichungen (SD) für EKG Vergleichswerte Parameter** | **M ± SD** |
|---|---|
| Kreuzkorrelation | 0.94 ± 0.05 |
| Mittlerer quadratischer Fehler (MSE) | 0.01 ± 0.01 |
| Normalisierter mittlerer quadratischer Fehler (NMSE) | 0.79 ± 0.59 |
| Effektivwert (RMS) | 0.84 ± 0.30 |
| normalisierter Effektivwert (NRMS) | 0.09 ± 0.05 |
| Prozent der Effektivwert-Abweichung | 84.4 ± 30.5 |

Um QRS-Komplexe und R-Spitzen in den Signalen zu identifizieren, wurde der Pan-Tompkins-Algorithmus angewendet. Die Anzahl der korrekt erkannten R-Spitzen sowie die Dauer der QRS-Komplexe wurden verwendet, um die Anzahl der R-Spitzen und die Dauer der QRS-Komplexe zu vergleichen. Um die Unterschiede zwischen dem EKGGT und dem EKGT zu untersuchen, wurde ein nicht-parametrischer Bland-Altman-Test durchgeführt. Diese Bland-Altman-Analyse zeigte hypothesenkonform keine signifikanten Abweichungen zwischen dem EKGGT und dem EKGT sowohl für die Anzahl der identifizierten R-Spitzen (mittlerer Bias = -8.0, 95%CI = -60 bis 44, r2=0.97, p=0.56) als auch für die Länge der QRS-Komplexe (mittlerer Bias = -0.34, 95%CI = -1.9 bis 1.2, r2=0.02, p=0.12).

### Bewertung durch Fachexperten

Hierfür wurden Rückmeldungen von 15 Kardiologen gesammelt, die sowohl Beispiele von kongruenten Signalen als auch von Signalen mit einer stärkeren statistischen Abweichung untersuchten. Die Experten wurden gebeten, den rhythmologischen und morphologischen diagnostischen Wert der Signale auf einer 5-Punkte-Likert-Skala zu bewerten (1 - sehr schlecht, 2 - schlecht, 3 - neutral, 4 - gut, 5 - sehr gut). Die durchschnittlichen Ergebnisse für die kongruenten Signale erreichten 4,87 von 5 für den rhythmologischen und 4,67 von 5 für den morphologischen diagnostischen Wert. Selbst bei den Signalen mit geringerer statistischer Kongruenz ergab das durchschnittliche Ergebnis 4,73 von 5 für den rhythmologischen und 4,60 von 5 für den morphologischen diagnostischen Wert. Diese Ergebnisse zeigen, dass das vorgeschlagene Verfahren die Bestimmung eines EKG-Signals 1 mit einer hohen Reliabilität und Validität sicherstellt. Auf der Signalebene konnten starke Korrelationen zwischen dem EKGT und dem EKGGT (Kreuzkorrelation r=0,94) gefunden werden. Auf Merkmalsebene belegen Vergleiche für die Anzahl der R-Spitzen und die Länge der QRS-Komplexe die Übereinstimmung des EKGGT mit dem durch die erfindungsgemäß angewendete Transformation bestimmten EKG-Signal 1. Im Allgemeinen lieferte der verwendete Datensatz eine hohe Qualität der SKG- und EKGGT-Signale. Dennoch enthielten einige Aufzeichnungen Bewegungsartefakte, die sich auf beide Signale auswirkten. Qualitativ minderwertige EKGGT-Daten und Bewegungsartefakte in den SKG-Daten vermindern die Qualität des durch Transformation bestimmten EKG-Signals 1 (EKGT), beeinflussen die Anzahl der erkannten R-Spitzen in verrauschten Signalabschnitten und senken den Korrelationskoeffizienten. Andererseits verringerten Artefakte im EKGGT auch den Korrelationskoeffizienten, wenn das artefaktfreie SKG-Signal 2 die Bestimmung eines qualitativ hochwertigen EKG-Signals 2 ermöglichte. In diesen Fällen erweist sich das wie erfindungsgemäß bestimmte EKG-Signal 1 als besser als die aufgezeichnete Grundwahrheit. Insbesondere im Falle von Artefakten im EKGGT-Signal durch falsche Elektrodenplatzierung kann das durch Transformation bestimmte EKG-Signal 1 genauere Ergebnisse liefern, da es unabhängig von einem Elektrodenanschluss bzw. einer korrekten Elektrodenplatzierung erzeugbar ist. Auch das systematische Feedback der Kardiologen belegt die klinische Validität und Relevanz.

Zusätzlich ermöglicht das erfindungsgemäß vorgeschlagene Verfahren, welches auch als Heart.Al-Verfahren bezeichnet werden kann, dass rhythmologische Pathologien (z.B. Vorhofflimmern) zuverlässig identifiziert werden können. Weiter ist die kontaktlose Anwendbarkeit des Verfahrens, dessen einfache Anwendung und die hohe Verfügbarkeit von Vorteil. Ebenfalls vorteilhaft ist die Möglichkeit, das Verfahren mit SKG-Erfassungseinrichtungen in Krankenhausbetten oder Betten in Pflegeeinrichtungen oder sogar in der häuslichen Umgebung anzuwenden. Auch vorteilhaft ist die leichte Verwendbarkeit in ländlichen Gebieten, in denen es oft an Allgemeinmedizinern und insbesondere an Fachärzten mangelt. Das vorgeschlagene Verfahren kann in einem solchen Szenario für telemedizinische Anwendungen einfach und kostengünstig eingesetzt werden.

Weiter kann ein existierendes Gerät mit einer zur Erfassung eines SKG-Signals 2 tauglichen Einrichtung, z.B. einem Beschleunigungssensor oder einem Gyroskop, durch ein Softwareupdate in die Lage versetzt werden, das vorgeschlagene Verfahren durchzuführen. Somit kann die von dem Verfahren bereitgestellte Funktionalität auf einer Vielzahl von Geräten nachgerüstet werden, was in eine breite Anwendbarkeit des Verfahrens gewährleistet. Ein weiterer Vorteil ist, dass eine einfache und zuverlässige dauerhafte Erfassung von präkordialen Bewegungen (SKG-Signal) möglich ist, die dann auch die dauerhafte und zuverlässige Bestimmung eines EKG-Signals ermöglicht, insbesondere in einem Zeitraum länger als 24 Stunden. Ebenfalls von Vorteil ist, dass die benötigte Sensorik kostengünstig ist und benötigte Sensoren in vielen nutzbaren Geräten bereits verbaut und daher -wie vorhergehend erläutert - für die Ausführung des Verfahrens genutzt werden können. Ebenfalls ist das vorgeschlagene Verfahren anwendbar, um nachträglich bereits erzeugte SKG-Signale 2 in EKG-Signale 1 zu transformieren. Dies ist insbesondere für wissenschaftlichen Untersuchungen interessant.

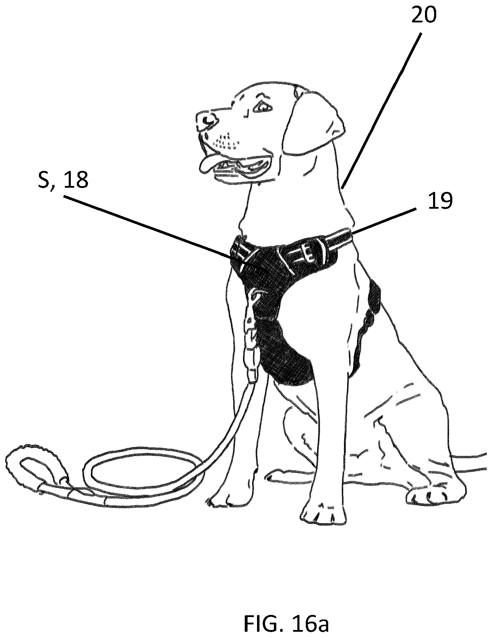

Fig. 16a zeigt eine schematische Darstellung eines Hundegurts 19 mit einer SKG-Erfassungseinrichtung S eines Systems 3 zur Erzeugung eines EKG-Signals 1 (siehe Fig. 1), wobei die SKG-Erfassungseinrichtung S als Beschleunigungssensor 18 ausgebildet ist. Dargestellt ist, dass die SKG-Erfassungseinrichtung S in einem Bereich des Hundegurts 19 angeordnet ist, der an einem Brustbereich des Hundes 20 anliegt, welcher den Hundegurt 19 in bestimmungsgemäßer Weise trägt.

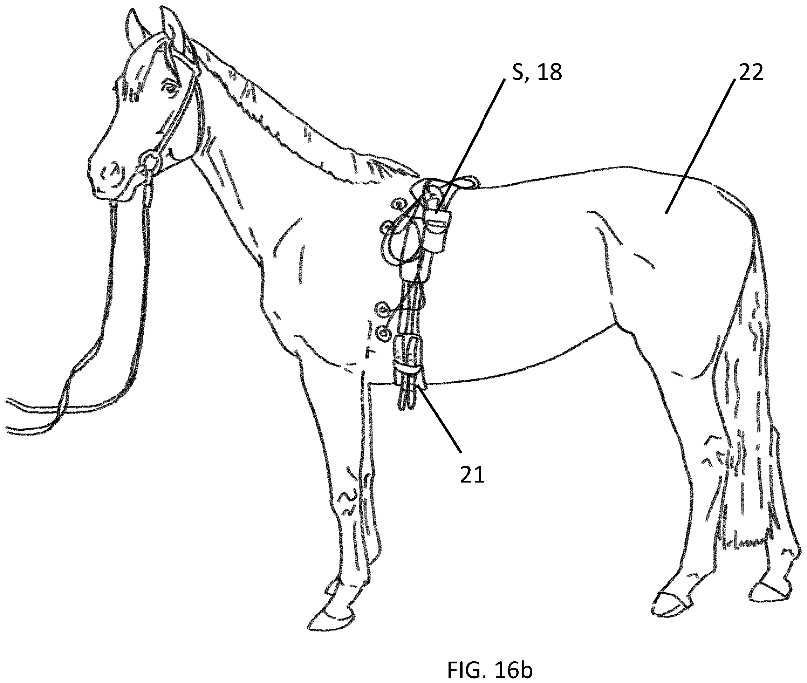

Fig. 16b zeigt eine schematische Darstellung eines Pferdehalfters 21 mit einer SKG-Erfassungseinrichtung S eines Systems 3 zur Erzeugung eines EKG-Signals 1 (siehe Fig. 1), wobei die SKG-Erfassungseinrichtung S als Beschleunigungssensor 18 ausgebildet ist. Dargestellt ist, dass die SKG-Erfassungseinrichtung S in einem Bereich des Halfters 21 angeordnet ist, der an einem oberen Rückenbereich des Pferdes 22 anliegt, welches das Halfter 21 in bestimmungsgemäßer Weise trägt. Es ist aber auch vorstellbar, die SKG-Erfassungseinrichtung S in einem Bereich des Halfters 21 anzuordnen, der an Bauch- oder Brustbereich des Pferdes 22 anliegt, welches das Halfter 21 in bestimmungsgemäßer Weise trägt.

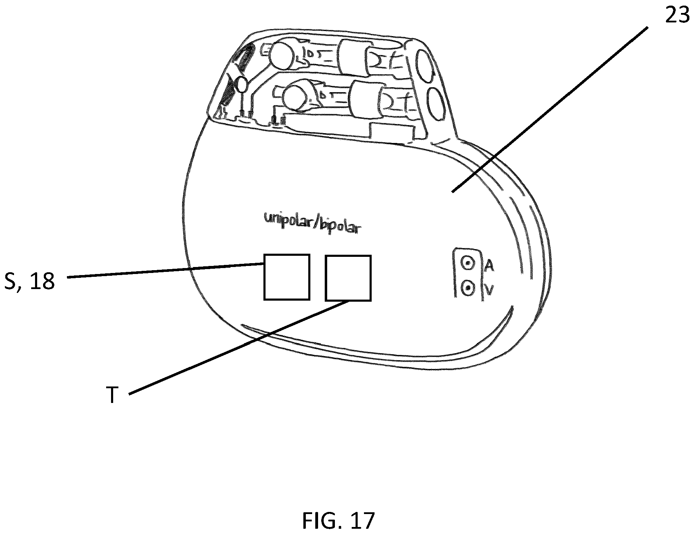

Fig. 17 eine schematische Darstellung eines Schrittmachers 23 mit einem System 3 zur Erzeugung eines EKG-Signals 1. Dargestellt ist ein ratenadaptiver Herzschrittmacher 23, der eine SKG-Erfassungseinrichtung S, die als Beschleunigungssensor 18 ausgebildet ist, umfasst. Weiter umfasst der Schrittmacher 22 eine Transformationseinrichtung T. Nicht dargestellt ist eine Kommunikationseinrichtung K des Schrittmachers 23, die das durch Transformation bestimmte EKG-Signal 1 an eine körperexterne Einrichtung, z.B. eine Anzeigeeinrichtung A oder eine Servereinrichtung 6, übertragen kann. Es ist allerdings nicht zwingend, dass der Schrittmacher 23 die Transformationseinrichtung T umfasst. So ist es auch möglich, dass der Schrittmacher 23 keine Transformationseinrichtung T umfasst und die Ausgangssignale (Rohsignale) der SKG-Erfassungseinrichtung S an eine schrittmacherexterne Recheneinrichtung übertragen werden, z.B. über die Kommunikationseinrichtung K.

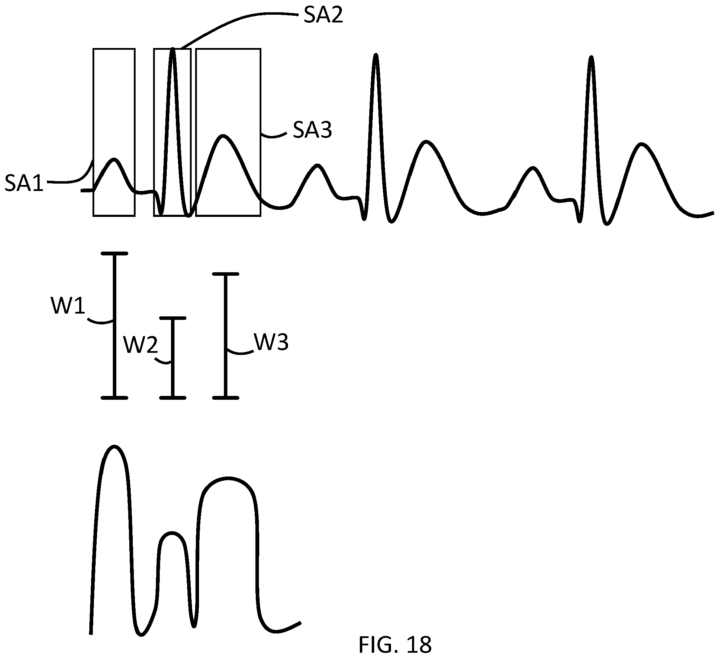

Fig. 18 zeigt eine exemplarische Darstellung von Gewichtungen verschiedener Signalabschnitte für die Auswertung einer Fehlerfunktion. In der oberen Zeile dargestellt ist ein EKG-Signal. In dem EKG-Signal sind drei verschiedene Signalabschnitte SA1, SA2, SA3 dargestellt, wobei die verschiedenen Signalabschnitte durch ein Rechteck eingefasst sind. Der erste Signalabschnitt SA1 ist ein P-Wellen-Signalabschnitt, der zweite Signalabschnitt SA2 ein QRS-Komplex-Signalabschnitt und der dritte Signalabschnitt SA3 ein T-Wellen-Signalabschnitt. Die zweite, mittlere Zeile zeigt Gewichtungsfaktoren w1, w2, w3, die den einzelnen Signalabschnitten SA1, SA2, SA3 zugeordnet sind. So ist dem ersten Signalabschnitt SA1 ein erster Gewichtungsfaktor w1, dem zweiten Signalabschnitt SA2 ein zweiter Gewichtungsfaktor w2 und dem dritten Signalabschnitt SA3 ein dritter Gewichtungsfaktor w3 zugeordnet. Erkenntlich ist, dass der erste Gewichtungsfaktor w1 größer als der zweite und der dritte Gewichtungsfaktor w2, w3 ist, wobei der dritte Gewichtungsfaktor w3 größer als der zweite Gewichtungsfaktor w2 ist. Es ist möglich, dass die Gewichtungsfaktoren größer als Eins sind. Es ist aber auch möglich, dass alle Gewichtungsfaktoren w1, w2, w3 gleich und größer als Eins sind, wodurch die für ein EKG relevanten Signalabschnitte SA1, SA2, SA3 im Verhältnis zu den verbleibenden, nicht relevanten Signalabschnitten höher gewichtet werden. Die dritte, untere Zeile zeigt einen Signalverlauf des gewichteten EKG-Signals, wobei die Amplitude des EKG-Signals im ersten Signalabschnitt SA1 mit dem ersten Gewichtungsfaktor w1, im zweiten Signalabschnitt SA2 mit dem zweiten Gewichtungsfaktor w2 und im dritten Signalabschnitt SA3 mit dem dritten Gewichtungsfaktor w3 gewichtet, insbesondere multipliziert, wurde. Auch kann die Gewichtung durch eine Faltung des EKG-Signals mit einer Fensterfunktion erfolgen. Durch diese Gewichtung kann insbesondere eine Amplitudenkompensation durchgeführt werden. Hierdurch kann vermieden werden, dass große Signaländerungen höher gewichtet werden als geringer Änderungen, was z.B. bei der Bestimmung der Abweichung mit dem Verfahren des mittleren quadrierten Fehlers der Fall ist. Im Falle des EKG-Signals beinhalten jedoch kleine Erhebungen (z.B. die in im ersten Signalabschnitt SA1 eingefasste P-Welle) wichtige Informationen. Es ist vorstellbar, dass auf diese Weise verschiedene Signalabschnitte eines durch Transformation bestimmten EKG-Signals 1 sowie verschiedene Signalabschnitte eines Referenz-EKG-Signals gewichtet werden und nach der Gewichtung dann die Abweichung zwischen den gewichteten Signalen bestimmt wird, um das Modell für die Transformation, insbesondere ein neuronales Netz, zu trainieren.

## Patentansprüche

1. Verfahren zur Erzeugung eines EKG-Signals (1), wobei mindestens ein herzbewegungsinduziertes Signal erfasst wird, wobei das mindestens eine erfasste herzbewegungsinduzierte Signal in mindestens ein EKG-Signal (1) transformiert wird, wobei das mindestens eine herzbewegungsinduzierte Signal ein SKG-Signal (2) ist, **dadurch gekennzeichnet, dass** durch die Transformation aus dem mindestens einen SKG-Signal (2) mehrere kanalspezifische Signale eines mehrkanaligen EKG-Signals bestimmt werden.

2. Verfahren nach Anspruch 1, **dadurch gekennzeichnet, dass** durch die Transformation aus dem mindestens einen SKG-Signal (2) alle kanalspezifischen Signale eines mehrkanaligen EKG-Signals bestimmt werden.

3. Verfahren nach einem der Ansprüche 1 bis 2, **dadurch gekennzeichnet, dass** die Transformation mittels eines Modells durchgeführt wird, welches durch maschinelles Lernen erzeugt wurde.

4. Verfahren nach Anspruch 3, **dadurch gekennzeichnet, dass** die Transformation mittels eines neuralen Netzes (NN) durchgeführt wird, welches insbesondere als Autoencoder oder als faltendes neuronales Netz oder als LSTM-Netz als neuronales Transformer-Netz ausgebildet ist.

5. Verfahren nach einem der Ansprüche 1 bis 3, **dadurch gekennzeichnet, dass** die Transformation mittels eines vorbestimmten mathematischen Modells oder mittels einer vorbestimmten Transformationsfunktion durchgeführt wird.

6. Verfahren nach einem der Ansprüche 3 bis 5, **dadurch gekennzeichnet, dass** zur Erzeugung des Modells eine Fehlerfunktion zur Bestimmung einer Abweichung zwischen einem durch Transformation bestimmten EKG-Signal (1) und einem Referenz-EKG-Signal ausgewertet wird, wobei bei der Auswertung der Fehlerfunktion verschiedene Signalabschnitte des durch Transformation bestimmten EKG-Signals (1) und/oder des Referenz-EKG-Signals und/oder der Abweichung verschieden gewichtet werden.

7. Verfahren nach einem der vorangegangenen Ansprüche, **dadurch gekennzeichnet, dass** das mindestens eine herzbewegungsinduzierte Signal berührungslos erfasst wird.

8. Verfahren nach einem der vorangegangenen Ansprüche, **dadurch gekennzeichnet, dass** das mindestens eine herzbewegungsinduzierte Signal vor der Transformation gefiltert und dann das gefilterte herzbewegungsinduziertes Signal in ein EKG-Signal (1) transformiert wird.

9. Verfahren nach einem der vorangegangenen Ansprüche, **dadurch gekennzeichnet, dass** das mindestens eine herzbewegungsinduzierte Signal von einer Erfassungseinrichtung eines Geräts (4) erzeugt wird, wobei die Transformation durch eine Recheneinrichtung (T) des Geräts (4) durchgeführt wird oder wobei das herzbewegungsinduzierte Signal an eine Recheneinrichtung (T) eines weiteren Geräts übertragen und die Transformation von dieser Recheneinrichtung (T) des weiteren Geräts durchgeführt wird.

10. Verfahren nach einem der vorangegangenen Ansprüche, **dadurch gekennzeichnet, dass** das mindestens eine herzbewegungsinduzierte Signal von einer Erfassungseinrichtung eines Geräts (4) erzeugt wird und das durch Transformation bestimmte EKG-Signal (1) auf einer Anzeigeeinrichtung (A) des Geräts (4) dargestellt wird oder wobei das mindestens eine herzbewegungsinduzierte Signal an eine Anzeigeeinrichtung eines weiteren Geräts übertragen und von dieser Anzeigeeinrichtung des weiteren Geräts dargestellt wird.

11. Verfahren nach einem der vorangegangenen Ansprüche, **dadurch gekennzeichnet, dass** vor der Transformation des mindestens einen herzbewegungsinduzierten Signals ein Funktionstest einer Erfassungseinrichtung durchgeführt wird, wobei das herzbewegungsinduzierte Signal nur dann transformiert wird, wenn eine Funktionsfähigkeit detektiert wird und/oder dass vor der Transformation des mindestens einen herzbewegungsinduzierten Signals eine Signalgüte des erfassten Signals bestimmt wird, wobei das herzbewegungsinduzierte Signal nur dann transformiert wird, wenn eine die Signalgüte größer als ein oder gleich einem vorbestimmtes/n Maß ist und/oder dass vor der Transformation des mindestens einen herzbewegungsinduzierten Signals eine Anordnung der Erfassungseinrichtung relativ zum Herz bestimmt wird, wobei das herzbewegungsinduzierte Signal nur dann transformiert wird, wenn die Anordnung einer vorbestimmten Anordnung entspricht oder weniger als ein vorbestimmtes Maß von dieser abweicht.

12. System zur Erzeugung eines EKG-Signals (1), wobei das System (3) mindestens eine Erfassungseinrichtung zur Erfassung mindestens eines herzbewegungsinduzierten Signals und mindestens eine Recheneinrichtung (T) umfasst, wobei das mindestens eine erfasste herzbewegungsinduzierte Signal mittels der Recheneinrichtung (T) in mindestens ein EKG-Signal (1) transformierbar ist, wobei das mindestens eine herzbewegungsinduzierte Signal ein SKG-Signal (2) ist, **dadurch gekennzeichnet, dass** durch die Transformation aus dem mindestens einen SKG-Signal (2) mehrere kanalspezifische Signale eines mehrkanaligen EKG-Signals bestimmbar sind.

13. System nach Anspruch 12, **dadurch gekennzeichnet, dass** die Erfassungseinrichtung in einem Inkubator (9), in einem Bett (13) oder in einem Fahrzeugsitz (17) oder in einen Herzschrittmacher oder in einen Tierbedarfsgegenstand integriert ist.

## Claims

1. Method for generating an ECG signal (1), wherein at least one cardiac motion-induced signal is detected, wherein the at least one detected cardiac motion-induced signal is transformed into at least one ECG signal (1), wherein the at least one cardiac motion-induced signal is an SCG signal (2), **characterized in that** a plurality of channel-specific signals of a multi-channel ECG signal are determined by the transformation from the at least one SCG signal (2).

2. Method according to claim 1, **characterized in that** all channel-specific signals of a multi-channel ECG signal are determined by the transformation from the at least one SKG signal (2).

3. Method according to any one of claims 1 to 2, **characterized in that** the transformation is carried out by means of a model generated by machine learning.

4. Method according to claim 3, **characterized in that** the transformation is carried out by means of a neural network (NN), which is designed in particular as an autoencoder or as a convolutional neural network or as an LSTM network as a neural transformer network.

5. Method according to any one of claims 1 to 3, **characterized in that** the transformation is performed by means of a predetermined mathematical model or by means of a predetermined transformation function.

6. Method according to one of claims 3 to 5, **characterized in that**, in order to generate the model, an error function for determining a deviation between an ECG signal (1) determined by transformation and a reference ECG signal is evaluated, wherein different signal sections of the ECG signal determined by transformation and/or of the reference ECG signal and/or of the deviation are weighted differently during the evaluation of the error function.

7. Method according to one of the preceding claims, **characterized in that** the at least one cardiac motion-induced signal is detected in a contact-free manner.

8. Method according to one of the preceding claims, **characterized in that** the at least one cardiac motion-induced signal is filtered before transformation and then the filtered cardiac motion-induced signal is transformed into an ECG signal (1).

9. Method according to one of the preceding claims, **characterized in that** the at least one cardiac motion-induced signal is generated by a n acquisition device of an apparatus (4), the transformation being carried out by a computing device (T) of the apparatus (4), or the cardiac motion-induced signal being transmitted to a computing device (T) of a further apparatus and the transformation being carried out by this computing device (T) of the further apparatus.

10. Method according to one of the preceding claims, **characterized in that** the at least one cardiac motion-induced signal is generated by a of an apparatus (4) and the ECG signal (1) determined by transformation is displayed on a display device (A) of the apparatus (4), or wherein the at least one cardiac motion-induced signal is transmitted to a display device of a further apparatus and is displayed by this display device of the further apparatus.

11. Method according to any one of the preceding claims, **characterized in that**, prior to the transformation of the at least one cardiac motion-induced signal, a functional test of a detection device is carried out, wherein the cardiac motion-induced signal is transformed only if a functional capability is detected and/or **in that**, prior to the transformation of the at least one cardiac motion-induced signal, a signal quality of the detected signal is determined, wherein the cardiac motion induced signal is transformed only if a the signal quality is greater than or equal to a predetermined measure(s) and/or that prior to the transformation of the at least one cardiac motion induced signal an arrangement of the detection device relative to the heart is determined, wherein the cardiac motion induced signal is transformed only if the arrangement is equal to a predetermined arrangement or deviates less than a predetermined amount therefrom.

12. System for generating an ECG signal (1), wherein the system (3) comprises at least one detection device for detecting at least one cardiac motion-induced signal and at least one computing device (T), wherein the at least one detected cardiac motion-induced signal is transformable into at least one ECG signal (1) by means of the computing device (T), wherein the at least one cardiac motion-induced signal is an SCG signal (2), **characterized in that** a plurality of channel-specific signals of a multi-channel ECG signal are determinable by the transformation from the at least one SCG signal (2)

13. System according to claim 12, **characterized in that** the detection device is integrated in an incubator (9), in a bed (13) or in a vehicle seat (17) or in a pacemaker or in an animal commodity

## Revendications

1. Procédé de génération d'un signal ECG (1), dans lequel au moins un signal induit par les mouvements cardiaques est détecté, dans lequel le au moins un signal induit par les mouvements cardiaques détecté est transformé en au moins un signal ECG (1), dans lequel le au moins un signal induit par les mouvements cardiaques est un signal SCG (2), **caractérisé en ce que** plusieurs signaux spécifiques aux canaux d'un signal ECG à plusieurs canaux sont déterminés par la transformation à partir du au moins un signal SCG (2).

2. Procédé selon la revendication 1, **caractérisé en ce que** tous les signaux spécifiques aux canaux d'un signal ECG à canaux multiples sont déterminés par la transformation à partir du au moins un signal SCG (2).

3. Procédé selon l'une des revendications 1 à 2, **caractérisé en ce que** la transformation est effectuée au moyen d'un modèle qui a été généré par apprentissage automatique.

4. Procédé selon la revendication 3, **caractérisé en ce que** la transformation est effectuée au moyen d'un réseau neuronal (NN) qui est conçu en particulier comme un auto-encodeur ou comme un réseau neuronal convolutif ou comme un réseau LSTM comme un réseau neuronal de transformation.

5. Procédé selon l'une des revendications 1 à 3, **caractérisé en ce que** la transformation est effectuée au moyen d'un modèle mathématique prédéterminé ou au moyen d'une fonction de transformation prédéterminée.

6. Procédé selon l'une des revendications 3 à 5, **caractérisé en ce que**, pour la production du modèle, une fonction d'erreur est évaluée pour déterminer un écart entre un signal ECG (1) déterminé par transformation et un signal ECG de référence, différentes sections de signal du signal ECG déterminé par transformation et/ou du signal ECG de référence et/ou de l'écart sont pondérés différemment étant pondérées differentement lors de l'évaluation de la fonction d'erreur.

7. Procédé selon l'une des revendications précédentes, **caractérisé en ce que** le au moins un signal induit par le mouvement cardiaque est détecté sans contact.

8. Procédé selon l'une des revendications précédentes, **caractérisé en ce que** le au moins un signal induit par les mouvements cardiaques est filtré avant la transformation, puis le signal induit par les mouvements cardiaques filtré est transformé en un signal ECG (1).

9. Procédé selon l'une des revendications précédentes, **caractérisé en ce que** le au moins un signal induit par les mouvements cardiaques est généré par un dispositif d'acquisition d'un appareil (4), la transformation étant effectuée par un dispositif de calcul (T) de l'appareil (4) ou le signal induit par les mouvements cardiaques étant transmis à un dispositif de calcul (T) d'un autre appareil et la transformation étant effectuée par ce dispositif de calcul (T) de l'autre appareil.

10. Procédé selon l'une des revendications précédentes, **caractérisé en ce que** le au moins un signal induit par les mouvements cardiaques est généré par un dispositif d'acquisition d'un appareil (4) et le signal ECG (1) déterminé par transformation est représenté sur un dispositif d'affichage (A) de l'appareil (4) ou le au moins un signal induit par les mouvements cardiaques est transmis à un dispositif d'affichage d'un autre appareil et est représenté par ce dispositif d'affichage de l'autre appareil.

11. Procédé selon l'une des revendications précédentes, **caractérisé en ce qu'**avant la transformation du au moins un signal induit par les mouvements cardiaques, un test de fonctionnement d'un dispositif de détection est effectué, le signal induit par les mouvements cardiaques n'étant transformé que si une capacité de fonctionnement est détectée et/ou **en ce qu'**avant la transformation du au moins un signal induit par les mouvements cardiaques, une qualité de signal du signal détecté est déterminée, le signal induit par les mouvements cardiaques n'étant transformé que si une qualité de signal est supérieure ou égale à une/des mesure(s) prédéterminée(s) et/ou **en ce qu'**avant la transformation du au moins un signal induit par les mouvements cardiaques, une disposition du dispositif de détection par rapport au coeur est déterminée, le signal induit par les mouvements cardiaques n'étant transformé que si la disposition correspond à une disposition prédéterminée ou s'écarte moins d'une mesure prédéterminée de celle-ci.

12. Système pour générer un signal ECG (1), le système (3) comprenant au moins un dispositif de détection pour détecter au moins un signal induit par les mouvements cardiaques et au moins un dispositif de calcul (T), le au moins un signal induit par les mouvements cardiaques détecté pouvant être transformé en au moins un signal ECG (1) au moyen du dispositif de calcul (T), le au moins un signal induit par les mouvements cardiaques étant un signal SCG (2), **caractérisé en ce que** plusieurs signaux spécifiques aux canaux d'un signal ECG à plusieurs canaux sont déterminables par la transformation à partir du au moins un signal SCG (2).

13. Système selon la revendication 12, **caractérisé en ce que** le dispositif de détection est intégré dans un incubateur (9), dans un lit (13) ou dans un siège de véhicule (17) ou dans un stimulateur cardiaque ou dans un accessoire pour animaux.
